(19) Europäisches Patentamt
European Patent Office
Office européen des brevets

(11) EP 2 828 779 B1

(12) **EUROPEAN PATENT SPECIFICATION**

(45) Date of publication and mention
of the grant of the patent:
**08.05.2019 Bulletin 2019/19**

(21) Application number: **13763804.5**

(22) Date of filing: **12.03.2013**

(51) Int Cl.:
***G06F 19/00*** *(2018.01)*       *G06F 19/26* *(2011.01)*

(86) International application number:
**PCT/CA2013/050183**

(87) International publication number:
**WO 2013/138923 (26.09.2013 Gazette 2013/39)**

(54) **SYSTEMS AND METHODS FOR MAKING TWO DIMENSIONAL GRAPHS OF MACROMOLECULES**

SYSTEME UND VERFAHREN ZUR HERSTELLUNG ZWEIDIMENSIONALER GRAFIKEN VON MAKROMOLEKÜLEN

SYSTÈMES ET PROCÉDÉS D'ÉTABLISSEMENT DE GRAPHIQUES BIDIMENSIONNELS DE MACROMOLÉCULES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.03.2012 US 201261613711 P**

(43) Date of publication of application:
**28.01.2015 Bulletin 2015/05**

(73) Proprietor: **Zymeworks Inc.
Vancouver, British Colombia V6H 3V9 (CA)**

(72) Inventors:
• **OHRN, Anders
Toronto, Ontario M4Y 1C5 (CA)**
• **MACDONALD, Scott Paul
Vancouver British Columbia V5Y 0C6 (CA)**

(74) Representative: **Grund, Martin
Grund Intellectual Property Group
Patentanwalt und Solicitor PartG mbB
Postfach 44 05 16
80754 München (DE)**

(56) References cited:
**EP-A2- 1 939 779        WO-A1-99/01744
WO-A1-2010/000268        WO-A1-2011/047684
US-A- 6 125 235          US-A1- 2004 088 116
US-A1- 2007 143 030**

• **RAYMOND J. W. ET AL: "Maximum common
subgraph isomorphism algorithms for the
matching of chemical structures", JOURNAL OF
COMPUTER-AIDED MOLECULAR DESIGN,
ESCOM SCIENCE PUBLISHERS BV, XX, vol. 16,
no. 7, 1 July 2002 (2002-07-01), pages 521-533,
XP002466663, ISSN: 0920-654X, DOI:
10.1023/A:1021271615909**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD**

The disclosed embodiments relate generally to systems and methods for visualizing complex molecules, such as polymers (e.g., proteins, nucleic acids, ribonucleic acids, polysaccharides, *etc.),* dendimers, organometallic complexes, surfactant self-assemblies and complex fullerenes in two dimensions.

**BACKGROUND**

In many applications, such as macromolecular structural studies, drug discovery, diagnostic development, detergent design, polymer chemistry, polymer physics, and polymer science, large volumes of physical data are acquired relating to (i) the physical properties of residues of complex molecules and (ii) physical properties shared between discrete groups of atoms, such as residues, in such complex molecules. Examples of the former physical properties include, but are not limited to, accessible surface area, solvent-excluded surface area, electrical charge, hydrophobicity, hydrophilicity, polarity, aromaticity, molecular weight and volume. Examples of the latter include physical properties include, but are not limited to, hydrogen bonds, close hydrogen bonds, carbon-carbon contacts, carbon-nitrogen contacts, carbon-oxygen contacts, carbon-sulfur contacts, $\pi$-$\pi$ interactions, and $\pi$-cation interactions.

Moreover, complex molecules typically have many discrete groups of atoms, termed particles herein, and adopt unique complex three-dimensional conformations. This makes visualization of the above-identified physical data challenging. US Patent No. 6,125,235 is directed to a computer-assisted method for generating a representation of a three dimensional structure of a molecule, but is based on the three-dimensional coordinates representing the molecule. WO 2011/047684 discloses predicting actual physical molecular structures using fatgraph modeling. WO 2010/000268 describes a computer-implemented method for visualizing a complex molecule in two dimensions based on complex molecule data, but lacks the application of a cost function algorithm for refining the values of coordinates in the set of the two-dimensional coordinates thus delivering a more precise molecule representation. Thus, given the above background, what is needed in the art are improved systems and methods for visualizing relational data associated with the physical properties of particles of complex molecules.

**SUMMARY**

Systems and methods for two-dimensional visualization of a complex molecule that address the shortcomings of the prior art are provided. In the present disclosure, the three-dimensional coordinates of the complex molecule are compressed into a two-dimensional graph with minimized loss in structural fidelity. The two-dimensional graph comprises nodes and edges. Each node corresponds to a part of the complex molecule. Edges between respective node pairs correspond to a physical property shared by the respective node pairs. More specifically, a characteristic of an edge between a pair of nodes is determined by a property shared by the portions of the complex molecule represented by the pair of nodes. For instance, if the pair of nodes represent portions of the complex molecule that are covalently bound to each other, the edge may be drawn as a thick dark line. Here, the characteristic then is the fact that the edge is drawn in this manner. In some embodiments, the complex molecule macromolecule comprising a nucleic acid or a protein and each node represents a residue in the macromolecule. In some embodiments, a characteristic of each node in the graph is determined by a physical property of the portion of the macromolecule that the node represents. For instance, in some embodiments, the physical property is hydrophobicity, with the nodes for more hydrophobic particles within the complex molecule being drawn larger than the nodes for more hydrophilic particles within the complex molecule. The disclosed systems and methods for making graphs produce graphs that are highly advantageous because they allow for the visualization of physical properties of complex molecules in two dimensions.

In one aspect, the present disclosure provides systems and methods for two-dimensional visualization of a complex molecule. The complex molecule comprises a set of particles $\{p_1, ..., p_N\}$. For instance, in some embodiments, each particle is a residue. In one particular example, the complex molecule is a protein and each particle in the set of particles is an amino acid residue of the protein. A set of $N$ three-dimensional coordinates $\{x_1, ..., x_N\}$ is obtained, each $x_i$ in $\{x_1, ..., x_N\}$ describing a three-dimensional position for a corresponding particle $p_i$ in $\{p_1, ..., p_N\}$. In typical embodiments, there is only one coordinate for each particle, although more than one coordinate is possible. It will be appreciated that each particle may comprise several covalently bound atoms and thus may have several coordinates, for instance, one for each atom. In some such embodiments, a single coordinate is selected for each particle. In the case of proteins in accordance with some embodiments, the coordinate of the $C_\alpha$ carbon is selected. In some embodiments, the coordinate that represents the center of mass of the particle is selected to represent the particle in the set of $N$ three-dimensional coordinates $\{x_1, ..., x_N\}$. It will be appreciated that the three-dimensional coordinates of the macromolecule may be in any reference frame so long as each particle is in the same reference frame.

In accordance with the systems and methods of the present disclosure, a cost function containing the error in the set of two-dimensional coordinates $(c_1, ..., c_N)$ is constructed. Each $c_i$ in $(c_1, ..., c_N)$ corresponds to a three-dimensional coordinate $x_i$ in $\{x_1, ..., x_N\}$. The three-dimensional coordinates are used to devise an initial set of the two-dimensional coordinates using, for instance, a dimension reduction scheme such as linear principal component analysis. Using the initial set of the two-dimensional coordinates as a starting point, this cost function is then minimized until an exit condition is achieved. The minimization alters the values of $(c_1, ..., c_N)$ and produces a refined set of two-dimensional coordinates that reproduces the three-dimensional structural features of the complex molecule in two-dimensional space with a reduced loss of structural fidelity.

With the optimized two-dimensional coordinates in hand, it is possible to construct the two-dimensional graph. Each respective optimized coordinate $c_i$ in $(c_1, ..., c_N)$ uniquely corresponds to (i) a particle in the complex molecule and (ii) a node in the graph. Each respective edge in the graph is bounded by a pair of nodes. Each respective edge is drawn in the graph in a manner that represents a physical characteristic shared by the pair nodes that bounds the respective edge. To this end, a set of physical properties $S_M$ is obtained, each $s_{i,j}$ in $S_M$ representing a physical property shared by a pair of particles $(p_i, p_j)$ in $\{p_1, ..., p_N\}$.

Advantageously, in addition to representing physical properties shared by pairs of particles in the complex molecule, physical properties of the particles themselves may be represented in the graph. To this end, a second set of physical properties $K_M$ is obtained. Each physical property $k_i$ in $K_M$ represents a physical property of a corresponding particle $p_i$ in $\{p_1, ..., p_N\}$. Then, a characteristic of a respective node in the plurality of nodes in the graph is determined by a value of or a type of the physical property of the corresponding particle $p_i$ in $K_M$.

## BRIEF DESCRIPTION OF THE DRAWINGS

Figure 1 is a block diagram illustrating a system, according to some embodiments.

Figure 2 illustrates a method for visualizing complex molecules in two dimensions, according to some embodiments.

Figure 3 illustrates a three dimensional representation of the Rab4 binding domain (PDB accession code 1YZM) consisting of two slightly tilted helices in contact, in accordance with the prior art.

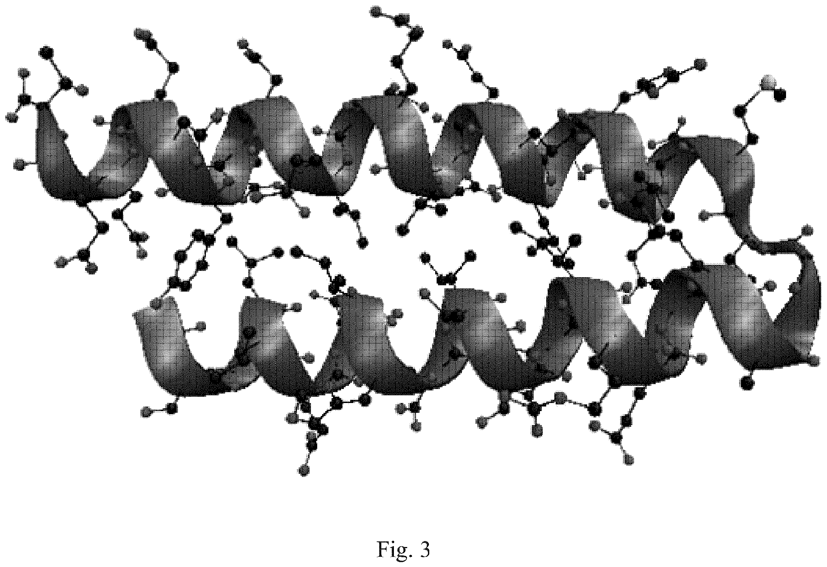

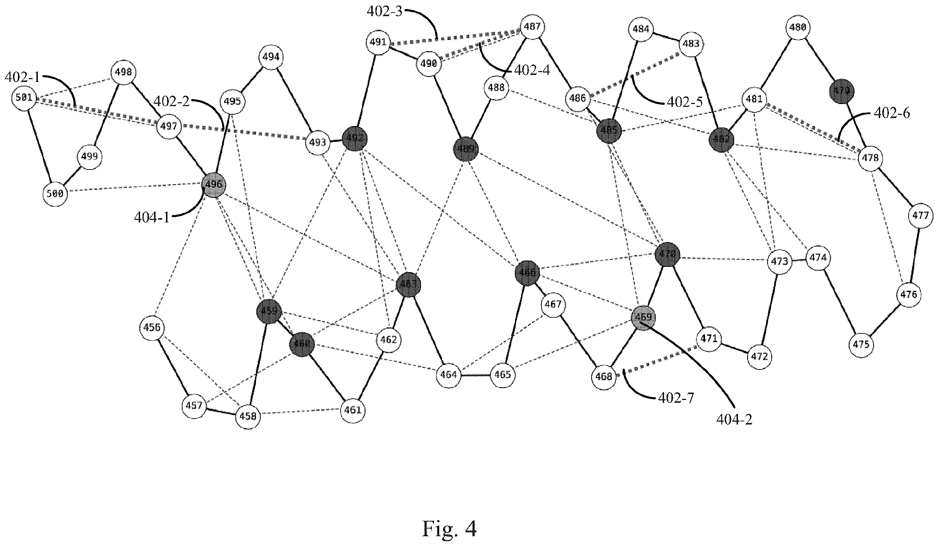

Figure 4 illustrates the Rab4 binding domain of Figure 3 rendered as a two dimensional graph with nodes and edges and conveying physical information about residues of the Rab4 binding domain in accordance with the systems and methods of the present disclosure. Solid lines connect residues that share a covalent peptide bond, thick dashed lines represent hydrogen bonds where at least one of the corresponding residue partners include a side-chain atom on the hydrogen bond, dashed lines represent carbon-carbon contacts, dark gray circles represent aliphatic residues, light gray circles represent aromatic residues, and white circles represent polar residues.

Figure 5 illustrates a three dimensional representation of the beta strand in accordance with the prior art.

Figure 6 illustrates the beta strand of Figure 5 rendered as a two dimensional graph with nodes and edges and conveying physical information about residues of the beta strand of Figure 5 in accordance with the systems and methods of the present disclosure.

## DETAILED DESCRIPTION OF THE EMBODIMENTS

The embodiments described herein provide systems and methods for visualizing macromolecules in two dimensions, according to the appended claims.

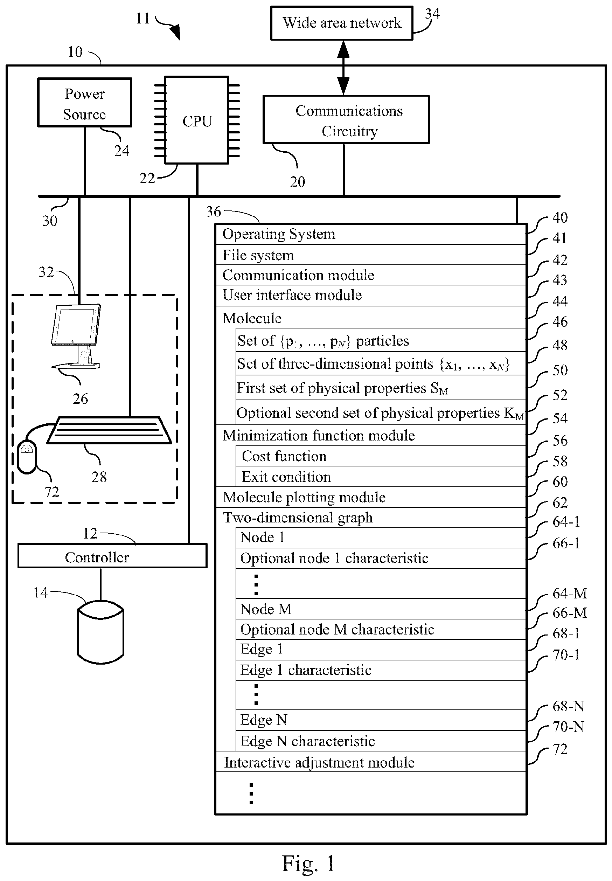

Figure 1 is a block diagram illustrating a computer according to some embodiments. The computer 10 typically includes one or more processing units (CPU's, sometimes called processors) 22 for executing programs (e.g., programs stored in memory 36), one or more network or other communications interfaces 20, memory 36, a user interface 32, which includes one or more input devices (such as a keyboard 28, mouse 72, touch screen, keypads, etc.) and one or more output devices such as a display device 26, and one or more communication buses 30 for interconnecting these components. The communication buses 30 may include circuitry (sometimes called a chipset) that interconnects and controls communications between system components.

Memory 36 includes high-speed random access memory, such as DRAM, SRAM, DDR RAM or other random access solid state memory devices; and typically includes non-volatile memory, such as one or more magnetic disk storage devices, optical disk storage devices, flash memory devices, or other non-volatile solid state storage devices.

Memory 36 optionally includes one or more storage devices remotely located from the CPU(s) 22. Memory 36, or alternately the non-volatile memory device(s) within memory 36, comprises a non-transitory computer readable storage medium. In some embodiments, the non-volatile components in memory 36 include one or more hard drives 14 controlled by one or more hard drive controllers 12. In some embodiments, memory 36 or the computer readable storage medium of memory 36 stores the following programs, modules and data structures, or a subset thereof:

- an operating system 40 that includes procedures for handling various basic system services and for performing hardware dependent tasks;

- a file system 41 for handling basic file I/O tasks;

- an optional communication module 42 that is used for connecting the computer 10 to other computers via the one or more communication interfaces 20 (wired or wireless) and one or more communication networks 34, such as the Internet, other wide area networks, local area networks, metropolitan area networks, and so on;

- an optional user interface module 43 that receives commands from the user via the input devices 28, 72, etc. and generates user interface objects in the display device 26;

- molecule data 44 for a complex molecule that is to be visualized in two dimensions;

- a minimization function module 54 for minimizing a cost function 56 that represents the error a two dimensional coordinate set for the complex molecule incurs in representing a three dimensional coordinate set for the complex molecule to be visualized, as described herein, until an exit condition 58 is achieved;

- a molecule plotting module 60 for plotting the two-dimensional coordinates, after minimization, as a two-dimensional graph 62 comprising nodes 64 and edges 68, where each node 64 in the graph 62 represents a portion of the complex molecule 44 and a characteristic of each respective edge 68 in the graph is determined by a physical property of the portions of the complex molecule 44 represented by the nodes 64 bounding the respective edge 68; and

- an interactive adjustment module 72 for manually adjusting positions of nodes and/or edges in the two-dimensional graph.

In some embodiments, the complex molecule data 44 for the complex molecule of interest includes a set of $\{p_1, ..., p_N\}$ particles 46. Each particle $p_i$ in the set of $\{p_1, ..., p_N\}$ particles represents a different plurality of covalently bound atoms in the macromolecule. By plurality of covalently bound atoms in the complex molecule, it is meant that each atom in the plurality of atoms is covalently bound to at least one other atom in the plurality of atoms. This is the case, for instance, in some exemplary embodiments where the complex molecule is a protein or nucleic acid and each particle is one or more residue of the protein or nucleic acid. Thus, in some embodiments, each particle $p_i$ in the set of particles $\{p_1, ..., p_N\}$ is for a different residue in the macromolecule. For example, consider the case in which the macromolecule is a protein with three hundred residues. In this example, each of the three hundred residues would be a particle $p_i$ in the set of $\{p_1, ..., p_N\}$ particles.

In some embodiments, the complex molecule of interest comprises between 2 and 5,000 particles, between 20 and 50,000 particles, more than 30 particles, more than 50 particles, or more than 100 particles. In some embodiments, a particle $p_i$ in the set of particles $\{p_1, ..., p_N\}$ for the complex molecule of interest comprises two or more atoms, three or more atoms, four or more atoms, five or more atoms, six or more atoms, seven or more atoms, eight or more atoms, nine or more atoms or ten or more atoms. In some embodiments, each particle $p_i$ in the set of particles $\{p_1, ..., p_N\}$ for the complex molecule of interest comprises two or more atoms, three or more atoms, four or more atoms, five or more atoms, six or more atoms, seven or more atoms, eight or more atoms, nine or more atoms or ten or more atoms. In some embodiments the complex molecule of interest has a molecular weight of 100 Daltons or more, 200 Daltons or more, 300 Daltons or more, 500 Daltons or more, 1000 Daltons or more, 5000 Daltons or more, 10,000 Daltons or more, 50,000 Daltons or more or 100,000 Daltons or more.

Moreover, in some embodiments, complex molecule data 44 further comprises a set of $N$ three-dimensional coordinates $\{x_1, ..., x_N\}$ 48, where each respective $x_i$ in $\{x_1, ..., x_N\}$ corresponds to a $p_i$ in $\{p_1, ..., p_N\}$ and represents the position of $p_i$ in three-dimensional space. For example, in some embodiments, the complex molecule is a protein, each $p_i$ in the set of $\{p_1, ..., p_N\}$ particles represents a residue in the protein, and each respective $x_i$ in $\{x_1, ..., x_N\}$ is the three-dimensional coordinates of the $C_\alpha$ carbon of the residue represented by the $p_i$ in the set of $\{p_1, ..., p_N\}$ particles that corresponds to the respective $x_i$. In other embodiments, each respective $x_i$ in $\{x_1, ..., x_N\}$ is the three-dimensional coordinates of the center of mass of the $p_i$ in the set of $\{p_1, ..., p_N\}$ particles. In some embodiments, the complex molecule is a protein, each $p_i$ in the set of $\{p_1, ..., p_N\}$ particles represents a residue in the protein, and each respective $x_i$ in $\{x_1, ..., x_N\}$ is the three-dimensional coordinates of a predetermined main chain atom (N, $C_\alpha$, C, or O) of the residue represented by the $p_i$ in the set of $\{p_1, ..., p_N\}$ particles that corresponds to the respective $x_i$.

In some embodiments, complex molecule data 44 further comprises a first set of physical properties $S_M$ 50. Each physical property $s_{i,j}$ in $S_M$ represents a physical property shared by a corresponding pair of particles $(p_i, p_j)$ in $\{p_1, ..., p_N\}$. An example of such a physical properties represented by $s_{i,j}$ for the corresponding pair of particles $(p_i, p_j)$ in $\{p_1, ..., p_N\}$ is a presence of a covalent bond between a first atom in the plurality of atoms represented by particle $p_i$ and a second atom in the plurality of atoms represented by particle $p_j$.

In some embodiments, complex molecule data 44 further comprises a second set of physical properties $K_M$ 52. Each physical property $k_i$ in $K_M$ represents a physical property of a corresponding particle $p_i$ in $\{p_1, ..., p_N\}$. Examples of such physical properties include, but are not limited to, an accessible surface area or solvent-excluded surface area of a plurality of atoms in the complex molecule represented by the corresponding particle $p_i$. Further examples of such physical properties include, but are not limited to, an electrical charge, hydrophobicity, hydrophilicity, polarity, aromaticity, molecular weight, or volume of the plurality of atoms in the complex molecule that are represented by the corresponding particle $p_i$.

In some embodiments, the programs or modules identified above correspond to sets of instructions for performing a function described above. The sets of instructions can be executed by one or more processors (*e.g.*, the CPUs 22). The above identified modules or programs (*e.g.*, sets of instructions) need not be implemented as separate software programs, procedures or modules, and thus various subsets of these programs or modules may be combined or otherwise re-arranged in various embodiments. In some embodiments, memory 36 stores a subset of the modules and data structures identified above. Furthermore, memory 36 may store additional modules and data structures not described above.

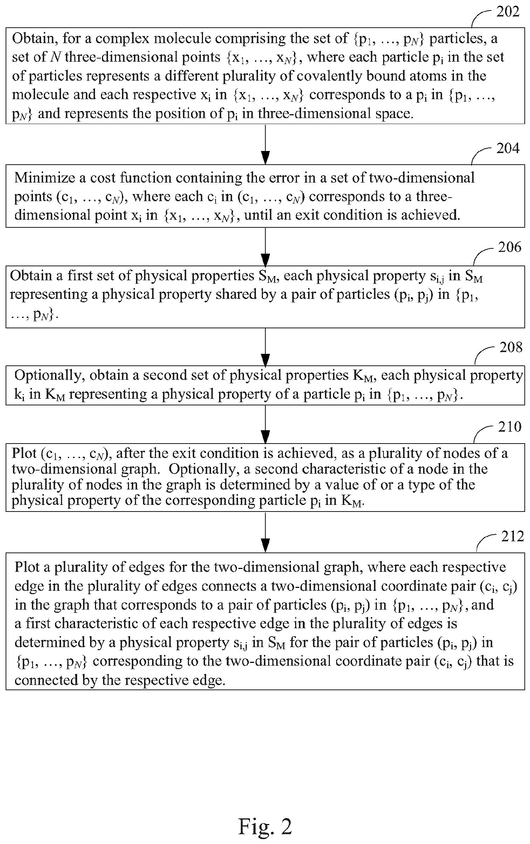

Now that a system in accordance with the systems and methods of the present disclosure has been described, attention turns to Figure 2 which illustrates an exemplary method in accordance with the present disclosure.

*Step 202.* In step 202, a set of *N* three-dimensional coordinates $\{x_1, ..., x_N\}$ 48 is obtained for a complex molecule comprising a set of $\{p_1, ..., p_N\}$ particles 46. Each particle $p_i$ in the set of $\{p_1, ..., p_N\}$ particles represents a different plurality of covalently bound atoms in the complex molecule. In one example, the complex molecule is a polynucleic acid and each particle $p_i$ in the set of $\{p_1, ..., p_N\}$ particles represents a nucleic acid residue in the polynucleic acid. In another example, the complex molecule is a polyribonucleic acid and each particle $p_i$ in the set of $\{p_1, ..., p_N\}$ particles represents a ribonucleic acid residue in the polyribonucleic acid. In still another example, the complex molecule is a polysaccharide and each particle $p_i$ in the set of $\{p_1, ..., p_N\}$ particles represents a monosaccharide unit or a disaccharide unit in the polysaccharide.

In still another example, the macromolecule is a protein and each particle $p_i$ in the set of $\{p_1, ..., p_N\}$ particles represents a residue in the protein. In some such embodiments, each respective $x_i$ in $\{x_1, ..., x_N\}$ is the three-dimensional coordinates of the $C_\alpha$ carbon of the residue represented by the $p_i$ in the set of $\{p_1, ..., p_N\}$ particles that corresponds to the respective $x_i$.

In still another example, the macromolecule is a protein or polypeptide and each particle $p_i$ in the set of $\{p_1, ..., p_N\}$ particles represents a residue in the protein polypeptide. In some such embodiments, each respective $x_i$ in $\{x_1, ..., x_N\}$ is the three-dimensional coordinate of the center of mass of the residue represented by the $p_i$ in the set of $\{p_1, ..., p_N\}$ particles that corresponds to the respective $x_i$.

In still another example, the complex molecule is a polymer and each particle $p_i$ in the set of $\{p_1, ..., p_N\}$ particles represents one or more different residues in the polymer. A polymer is a large molecule composed of repeating structural units. These repeating structural units are termed particles herein. In some embodiments, each particle $p_i$ in the set of $\{p_1, ..., p_N\}$ particles represents a single different residue in the polymer. To illustrate, consider the case where the polymer comprises 100 residues. In this instance, the set of $\{p_1, ..., p_N\}$ comprises 100 particles, with each particle in $\{p_1, ..., p_N\}$ representing a different one of the 100 particles. In another example, in some embodiments, each particle $p_i$ in the set of $\{p_1, ..., p_N\}$ particles represents a pair of particles in the polymer. In this instance, the set of $\{p_1, ..., p_N\}$ comprises 50 particles, with each particle in $\{p_1, ..., p_N\}$ representing a different one of the 50 particles. In some embodiments, the polymer is a natural material. In some embodiments, the polymer is a synthetic material. In some embodiments, the polymer is an elastomer, shellac, amber, natural or synthetic rubber, cellulose, Bakelite, nylon, polystyrene, polyethylene, polypropylene, or polyacrylonitrile, polyethylene glycol, or polysaccharide.

In some embodiments, the complex molecule is a heteropolymer (copolymer). A copolymer is a polymer derived from two (or more) monomeric species, as opposed to a homopolymer where only one monomer is used. Copolymerization refers to methods used to chemically synthesize a copolymer. Examples of copolymers include, but are not limited to, ABS plastic, SBR, nitrile rubber, styrene-acrylonitrile, styreneisoprene-styrene (SIS) and ethylene-vinyl acetate. Since a copolymer consists of at least two types of constituent units (also structural units, or particles), copolymers can be classified based on how these units are arranged along the chain. These include alternating copolymers with regular alternating A and B units. See, for example, Jenkins, 1996, "Glossary of Basic Terms in Polymer Science," Pure Appl.

Chem. 68 (12): 2287-2311, which is hereby incorporated herein by reference in its entirety. Additional examples of copolymers are periodic copolymers with A and B units arranged in a repeating sequence (e.g. (A-B-A-B-B-A-A-A-A-B-B-B)$_n$). Additional examples of copolymers are statistical copolymers in which the sequence of monomer residues in the copolymer follows a statistical rule. If the probability of finding a given type monomer residue at a particular point in the chain is equal to the mole fraction of that monomer residue in the chain, then the polymer may be referred to as a truly random copolymer. See, for example, Painter, 1997, Fundamentals of Polymer Science, CRC Press, 1997, p 14, which is hereby incorporated by reference herein in its entirety. Still other examples of copolymers are block copolymers comprising two or more homopolymer subunits linked by covalent bonds. The union of the homopolymer subunits may require an intermediate non-repeating subunit, known as a junction block. Block copolymers with two or three distinct blocks are called diblock copolymers and triblock copolymers, respectively.

In some embodiments, the complex molecule of interest is in fact a plurality of polymers, where the polymers in the plurality of polymers do not all have the same molecular weight. In such embodiments, the polymers in the plurality of polymers fall into a weight range with a corresponding distribution of chain lengths. In some embodiments, the polymer is a branched polymer molecule comprising a main chain with one or more substituent side chains or branches. Types of branched polymers include, but are not limited to, star polymers, comb polymers, brush polymers, dendronized polymers, ladders, and dendrimers. See, for example, Rubinstein et al., 2003, Polymer physics, Oxford; New York: Oxford University Press. p. 6, which is hereby incorporated by reference herein in its entirety.

In some embodiments, the complex molecule of interest is a polypeptide. As used herein, the term "polypeptide" means two or more amino acids or residues linked by a peptide bond. The terms "polypeptide" and "protein" are used interchangeably and include oligopeptides and peptides. An "amino acid," "residue" or "peptide" refers to any of the twenty standard structural units of proteins as known in the art, which include imino acids, such as proline and hydroxyproline. The designation of an amino acid isomer may include D, L, R and S. The definition of amino acid includes nonnatural amino acids. Thus, selenocysteine, pyrrolysine, lanthionine, 2-aminoisobutyric acid, gamma-aminobutyric acid, dehydroalanine, ornithine, citrulline and homocysteine are all considered amino acids. Other variants or analogs of the amino acids are known in the art. Thus, a polypeptide may include synthetic peptidomimetic structures such as peptoids. See Simon et al., 1992, Proceedings of the National Academy of Sciences USA, 89, 9367, which is hereby incorporated by reference herein in its entirety. See also Chin et al., 2003, Science 301, 964; and Chin et al., 2003, Chemistry & Biology 10, 511, each of which is incorporated by reference herein in its entirety.

A polypeptide may also have any number of posttranslational modifications. Thus, a polypeptide includes those that are modified by acylation, alkylation, amidation, biotinylation, formylation, γ-carboxylation, glutamylation, glycosylation, glycylation, hydroxylation, iodination, isoprenylation, lipoylation, cofactor addition (for example, of a heme, flavin, metal, *etc.),* addition of nucleosides and their derivatives, oxidation, reduction, pegylation, phosphatidylinositol addition, phosphopantetheinylation, phosphorylation, pyroglutamate formation, racemization, addition of amino acids by tRNA (for example, arginylation), sulfation, selenoylation, ISGylation, SUMOylation, ubiquitination, chemical modifications (for example, citrullination and deamidation), and treatment with other enzymes (for example, proteases, phosphotases and kinases). Other types of posttranslational modifications are known in the art and are also included.

In some embodiments, the complex molecule of interest is an organometallic complex. An organometallic complex is chemical compound containing bonds between carbon and metal. In some instances, organometallic compounds are distinguished by the prefix "organo-" *e.g.* organopalladium compounds. Examples of such organometallic compounds include all Gilman reagents, which contain lithium and copper. Tetracarbonyl nickel, and ferrocene are examples of organometallic compounds containing transition metals. Other examples include organomagnesium compounds like iodo(methyl)magnesium MeMgI, diethylmagnesium (Et$_2$Mg), and all Grignard reagents; organolithium compounds such as n-butyllithium (n-BuLi), organozinc compounds such as diethylzinc (Et$_2$Zn) and chloro(ethoxycarbonyl-methyl)zinc (ClZ$_n$CH$_2$C(=O)OEt); and organocopper compounds such as lithium dimethylcuprate (Li$^+$[CuMe$_2$]$^-$). In addition to the traditional metals, lanthanides, actinides, and semimetals, elements such as boron, silicon, arsenic, and selenium are considered form organometallic compounds, e.g. organoborane compounds such as triethylborane (Et$_3$B).

In some embodiments, the complex molecule of interest is a surfactant. Surfactants are compounds that lower the surface tension of a liquid, the interfacial tension between two liquids, or that between a liquid and a solid. Surfactants may act as detergents, wetting agents, emulsifiers, foaming agents, and dispersants. Surfactants are usually organic compounds that are amphiphilic, meaning they contain both hydrophobic groups (their tails) and hydrophilic groups (their heads). Therefore, a surfactant molecule contains both a water insoluble (or oil soluble) component and a water soluble component. Surfactant molecules will diffuse in water and adsorb at interfaces between air and water or at the interface between oil and water, in the case where water is mixed with oil. The insoluble hydrophobic group may extend out of the bulk water phase, into the air or into the oil phase, while the water soluble head group remains in the water phase. This alignment of surfactant molecules at the surface modifies the surface properties of water at the water/air or water/oil interface.

Examples of ionic surfactants include ionic surfactants such as anionic, cationic, or zwitterionic (ampoteric) surfactants. Anionic surfactants include (i) sulfates such as alkyl sulfates (*e.g.*, ammonium lauryl sulfate, sodium lauryl sulfate), alkyl ether sulfates (*e.g.*, sodium laureth sulfate, sodium myreth sulfate), (ii) sulfonates such as docusates (*e.g.,* dioctyl sodium sulfosuccinate), sulfonate fluorosurfactants (*e.g.*, perfluorooctanesulfonate and perfluorobutanesulfonate), and alkyl benzene sulfonates, (iii) phosphates such as alkyl aryl ether phosphate and alkyl ether phosphate , and (iv) carboxylates such as alkyl carboxylates (*e.g.*, fatty acid salts (soaps) and sodium stearate), sodium lauroyl sarcosinate, and carboxylate fluorosurfactants (*e.g.*, perfluorononanoate, perfluorooctanoate, *etc.*). Cationic surfactants include pH-dependent primary, secondary, or tertiary amines and permanently charged quaternary ammonium cations. Examples of quaternary ammonium cations include alkyltrimethylammonium salts (*e.g.,* cetyl trimethylammonium bromide, cetyl trimethylammonium chloride), cetylpyridinium chloride (CPC), benzalkonium chloride (BAC), benzethonium chloride (BZT), 5-bromo-5-nitro-1,3-dioxane, dimethyldioctadecylammonium chloride, and dioctadecyldimethylammonium bromide (DODAB) . Zwitterionic surfactants include sulfonates such as CHAPS (3-[(3-Cholamidopropyl)dimethylammonio]-1-propanesulfonate) and sultaines such as cocamidopropyl hydroxysultaine. Zwitterionic surfactants also include carboxylates and phosphates.

Nonionic surfactants include fatty alcohols such as cetyl alcohol, stearyl alcohol, cetostearyl alcohol, and oleyl alcohol. Nonionic surfactants also include polyoxyethylene glycol alkyl ethers (*e.g.*, octaethylene glycol monododecyl ether, pentaethylene glycol monododecyl ether), polyoxypropylene glycol alkyl ethers, glucoside alkyl ethers (decyl glucoside, lauryl glucoside, octyl glucoside, *etc.*), polyoxyethylene glycol octylphenol ethers ($C_8H_{17}$-($C_6H_4$)-(O-$C_2H_4$)$_{1-25}$-OH), polyoxyethylene glycol alkylphenol ethers ($C_9H_{19}$-($C_6H_4$)-(O-$C_2H_4$)$_{1-25}$-OH, glycerol alkyl esters (*e.g.*, glyceryl laurate), polyoxyethylene glycol sorbitan alkyl esters, sorbitan alkyl esters, cocamide MEA, cocamide DEA, dodecyldimethylamine oxideblock copolymers of polyethylene glycol and polypropylene glycol (poloxamers), and polyethoxylated tallow amine . In some embodiments, the complex molecule is a reverse micelle, or liposome.

In some embodiments, the complex molecule is a fullerene. A fullerene is any molecule composed entirely of carbon, in the form of a hollow sphere, ellipsoid or tube. Spherical fullerenes are also called buckyballs, and they resemble the balls used in association football. Cylindrical ones are called carbon nanotubes or buckytubes. Fullerenes are similar in structure to graphite, which is composed of stacked graphene sheets of linked hexagonal rings; but they may also contain pentagonal (or sometimes heptagonal) rings.

In some embodiments, the set of *N* three-dimensional coordinates $\{x_1, ..., x_N\}$ 48 for the complex molecule of interest are obtained by x-ray crystallography, nuclear magnetic resonance spectroscopic techniques, or electron microscopy. In some embodiments, the set of *N* three-dimensional coordinates $\{x_1, ..., x_N\}$ is obtained by modeling (*e.g.*, molecular dynamics simulations).

In some embodiments, the complex molecule is a macromolecule and each particle $p_i$ in the set of $\{p_1, ..., p_N\}$ particles represents more than one residue of the macromolecule. For instance, in some embodiments, each particle represents two residues of the macromolecule. In some embodiments, each particle represents three residues of the macromolecule. In some embodiments, each particle represents four residues of the macromolecule. In some embodiments, the macromolecule includes two different types of polymers, such as a nucleic acid bound to a polypeptide. In some embodiments, the macromolecule includes two polypeptides bound to each other. In some embodiments, the macromolecule includes one or more metal ions (*e.g.* a metalloproteinase with one or more zinc atoms) and/or is bound to one or more organic small molecules (*e.g.*, an inhibitor). In such instances, the metal ions and or the organic small molecules may be represented as one or more additional particles $p_i$ in the set of $\{p_1, ..., p_N\}$ particles representing the macromolecule.

In some embodiments, there are ten or more, twenty or more, thirty or more, fifty or more, one hundred or more, between one hundred and one thousand, or less than 500 particles in the complex molecule.

There is no requirement that each atom in a particle $p_i$ be covalently bound to each other atom in the particle. More typically, each atom in a particle $p_i$ is covalently bound to at least one other atom in the particle, as is the typical case in an amino acid residue in a polypeptide. Moreover, typically, for each respective particle $p_i$ in the set of $\{p_1, ..., p_N\}$ particles, there is at least one atom in the respective particle $p_i$ that is covalently bound to an atom in another particle in the set of $\{p_1, ..., p_N\}$ particles.

*Step 204.* In step 204, a cost function containing the error in a set of two-dimensional coordinates $(c_1, ..., c_N)$, where each $c_i$ in $(c_1, ..., c_N)$ corresponds to a three-dimensional coordinate $x_i$ in $\{x_1, ..., x_N\}$, is defined. Once the cost-function has been defined, the next step is to minimize it with respect to the two-dimensional coordinates $(c_1, ..., c_N)$. To perform such minimization, an initial configuration for the two-dimensional coordinates $(c_1, ..., c_N)$ is obtained. In some embodiments, an initial configuration for the two-dimensional coordinates $(c_1, ..., c_N)$ is obtained by applying a linear principal component analysis to the three-dimensional coordinates $\{x_1, ..., x_N\}$. In general, an initial configuration for the two-dimensional coordinates $(c_1, ..., c_N)$ can be obtained by applying any form of dimension reduction algorithm to the three-dimensional coordinates $\{x_1, ..., x_N\}$.

In some embodiments, the cost function has the form:

$$E(c_1, c_2, \ldots, c_N) = \sum_{i<j}^{N} w_{ij} \left| \delta_{ij} - D(c_i, c_j) \right|^2$$

where,

$i$ and $j$ are integers greater than zero,

$\delta_{ij}$ is a distance between a pair of three-dimensional coordinates $x_i$ and $x_j$ in $\{x_1, \ldots, x_N\}$,

$E(c_1, c_2, \ldots, c_N)$ is an error in the set of two-dimensional coordinates $(c_1, \ldots, c_N)$, where each two-dimensional coordinate $c_i$ in $(c_1, \ldots, c_N)$ uniquely corresponds to a three-dimensional coordinate $x_i$ in $\{x_1, \ldots, x_N\}$ so that each respective $p_i$ in $\{p_1, \ldots, p_N\}$ is represented by a three-dimensional coordinate $x_i$ in $\{x_1, \ldots, x_N\}$ and a corresponding two-dimensional coordinate $c_i$ in $(c_1, \ldots, c_N)$,

$D(c_i, c_j)$ is a distance between the two-dimensional coordinates $c_i$ and $c_j$ in $(c_1, \ldots, c_N)$, and

$w_{ij}$ is a weight for the two-dimensional pair $(p_i, p_j)$ in a matrix of weights, where the matrix of weights has a weight for each two-dimensional pair $(p_i, p_j)$ in $(p_1, \ldots, p_N)$.

In an embodiment in which Sammon mapping is used, the weights are defined as:

$$w_{ij} = \frac{1}{\delta_{ij}} \frac{1}{\sum_{k<l}^{N} \delta_{kl}}$$

where $\delta_{kl}$ is a distance between a pair of three-dimensional coordinates $x_k$ and $x_l$ in $\{x_1, \ldots, x_N\}$, While not intending to be limited by any particular theory, a justification for such weighting according to this formulation is that the separation between two particles that are close in the high-dimensional space will be given a greater weight. Hence, according to this proposed justification, local topology is better preserved than distal particle separations, which often is a desired property.

Once the cost function has been defined and an initial configuration for the two-dimensional coordinates $(c_1, \ldots, c_N)$ determined, any of a range of methods can be used to minimize the cost function until an exit condition is achieved. In some embodiments, the cost function is minimized by steepest decent. When steepest decent minimization is used, derivatives of the cost function are calculated. The derivative of the cost function is derived as follows:

$$\frac{\partial E}{\partial c_m} = \frac{1}{\sum_{k<l}^{N} \delta_{kl}} \sum_{i<j}^{N} \frac{1}{\delta_{ij}} \frac{\partial}{\partial c_m} \left| \delta_{ij} - D(c_i, c_j) \right|^2 =$$

$$\frac{1}{\sum_{k<l}^{N} \delta_{kl}} \sum_{j, j \neq m}^{N} \frac{1}{\delta_{mj}} \frac{\partial}{\partial c_m} \left| \delta_{mj} - D(c_m, c_j) \right|^2 =$$

$$\frac{-2}{\sum_{k<l}^{N} \delta_{kl}} \sum_{j, j \neq m}^{N} \frac{1}{\delta_{mj}} \left| \delta_{mj} - D(c_m, c_j) \right| \frac{\partial}{\partial c_m} D(c_m, c_j) = \frac{-2}{\sum_{k<l}^{N} \delta_{kl}} \sum_{j, j \neq m}^{N} \frac{1}{\delta_{mj}} \left| \delta_{mj} - \right.$$

$$\left. D(c_m, c_j) \right| \frac{(c_m - c_j)}{D(c_m, c_j)}.$$

where $k, N, l, m, i, j$ are integers greater than zero.

The second equality follows from the observation that derivatives are zero for any distance not involving the particle $m$. The third equality follows from the chain-rule. The third equality follows from the derivative of the Euclidian distance between particle $m$ and $j$ in a two-dimensional space:

$$D(c_i, c_j) = \sqrt{\left( c_i^x - c_j^x \right)^2 + \left( c_i^y - c_j^y \right)^2}$$

where the superscript denotes the x- and y-component of the particle coordinate.

In some embodiments, the cost function is minimized using a quasi-Newton method, such as the Broyden-Fletcher-Goldfarb-Shanno (BFGS), which also only requires the above identified derivative. In quasi-Newton methods, the Hessian matrix of second derivatives need not be evaluated directly. Instead, the Hessian matrix is approximated using rank-one updates specified by gradient evaluations (or approximate gradient evaluations). Quasi-Newton methods are a generalization of the secant method to find the root of the first derivative for multidimensional problems. In multi-dimensions the secant equation does not specify a unique solution, and quasi-Newton methods differ in how they constrain the solution.

In some embodiments, the cost function is minimized using a random walk method, such as simulated annealing ("SA"), that does not require derivatives. For applications involving on the order of a few hundred particles a "hill-climbing method", such as steepest decent or BFGS, is expected to be optimal. The SA method is computationally more expensive. For a very large number of particles simulated annealing may be a better minimization technique than the hill-climbing methods.

As noted above, the cost function is minimized until an exit condition is achieved. In some instances, the exit condition is determined by the method by which the cost function is minimized. For example, Berinde, 1997, Novi SAD J. Math, 27, 19-26, which is incorporated herein by reference, outlines some exit conditions for Newton's method. In some embodiments, the exit condition is achieved when a predetermined maximum number of iterations of the refinement algorithm have been computed. In some embodiments, the predetermined maximum number of iterations is ten iterations, twenty iterations, one hundred iterations or one thousand iterations. For a given iteration n, where n is other than the first iteration the starting two-dimensional coordinates $(c_1, ..., c_N)$ are the two-dimensional coordinates $(c_1, ..., c_N)$ from the $n-1^{th}$ iteration. As discussed above, for the initial run of the refinement method on the initial two-dimensional coordinates $(c_1, ..., c_N)$, the two-dimensional coordinates $(c_1, ..., c_N)$ that were derived directly from the three dimensional coordinates $\{x_1, ..., x_N\}$ is used.

*Step 206.* Minimization of the cost function results in a refined set of two-dimensional coordinates $(c_1, ..., c_N)$ that represent the three dimensional coordinates of the complex molecule. Steps 206 through 212 of the method are advantageously directed to using this refined set of two-dimensional coordinates $(c_1, ..., c_N)$ to visualize physical properties of the complex molecule.

In step 206, a first set of physical properties $S_M$ is obtained. Each physical property $s_{i,j}$ in $S_M$ represents a physical property shared by a pair of particles $(p_i, p_j)$ in $\{p_1, ..., p_N\}$.

In some embodiments, the physical property represented by $s_{i,j}$ for the corresponding pair of particles $(p_i, p_j)$ in $\{p_1, ..., p_N\}$ is a presence of a covalent bond between a first atom in the plurality of atoms represented by particle $p_i$ and a second atom in the plurality of atoms represented by particle $p_j$, where i does not equal j. An example of such a covalent bond arises in the case where the pair of particles $(p_i, p_j)$ represent a first cysteine $(p_i)$ and a second cysteine $(p_j)$ and the two cysteines form a disulphide bond.

In some embodiments, the physical property represented by $s_{i,j}$ for the corresponding pair of particles $(p_i, p_j)$ in $\{p_1, ..., p_N\}$ is a presence of a hydrogen bond between a first atom in the plurality of atoms represented by particle $p_i$ and a second atom in the plurality of atoms represented by particle $p_j$. Hydrogen bonds are formed when an electronegative atom approaches a hydrogen atom bound to another electro-negative atom. The most common electro negative atoms in biochemical systems are oxygen (3.44) and nitrogen (3.04) while carbon (2.55) and hydrogen (2.22) are relatively electropositive. The hydrogen is normally covalently attached to one atom, the donor, but interacts electrostatically with the other, the acceptor. This interaction is due to the dipole between the electronegative atoms and the proton. Thus, the first atom in the plurality of atoms represented by particle $p_i$ is the donor and the second atom in the plurality of atoms represented by particle $p_j$ is the acceptor of the hydrogen, or vice versa. Moreover, the first atom in the plurality of atoms represented by particle $p_i$ and the second atom in the plurality of atoms represented by particle $p_j$ share the same hydrogen. The occurrence of hydrogen bonds in protein structures has been extensively reviewed by Baker & Hubbard, 1984, Prog. Biophy. Mol. Biol., 44, 97-179, which is hereby incorporated by reference herein in its entirety.

In some embodiments, the physical property represented by $s_{i,j}$ for the corresponding pair of particles $(p_i, p_j)$ in $\{p_1, ..., p_N\}$ is a presence of a carbon-carbon contact, a carbon-sulfur contact, or a sulfur-sulfur contact between a first atom in the plurality of atoms represented by particle $p_i$ and a second atom in the plurality of atoms represented by particle $p_j$. In some embodiments, a carbon-carbon contact, a carbon-sulfur contact, or a sulfur-sulfur contact occurs when the first atom and the second atom are each independently carbon or sulfur and the first atom and the second atom are within a predetermined distance of each other in the complex molecule. In some embodiments, this predetermined distance is 4.5 Angstroms. In some embodiments, this predetermined distance is 4.0 Angstroms.

In some embodiments, the physical property represented by $s_{i,j}$ for the corresponding pair of particles $(p_i, p_j)$ in $\{p_1, ..., p_N\}$ is a presence of a carbon-nitrogen contact between a first atom in the plurality of atoms represented by particle $p_i$ and a second atom in the plurality of atoms represented by particle $p_j$. In some embodiments, a carbon-nitrogen contact occurs when the first atom is a carbon and the second atom is a nitrogen and the first atom and the second atom are within a predetermined distance of each other in the complex molecule as defined by the three-dimensional coordinates $\{x_1, ..., x_N\}$, In some embodiments, this predetermined distance is 4.5 Angstroms. In some embodiments, this predetermined distance is 4.0 Angstroms. In some embodiments, this predetermined distance is 3.5 Angstroms.

In some embodiments, the physical property represented by $s_{i,j}$ for the corresponding pair of particles $(p_i, p_j)$

in $\{p_1, ..., p_N\}$ is a presence of a carbon-oxygen contact between a first atom in the plurality of atoms represented by particle $p_i$ and a second atom in the plurality of atoms represented by particle $p_j$. In some embodiments, a carbon-oxygen contact occurs when the first atom is a carbon and the second atom is a oxygen and the first atom and the second atom are within a predetermined distance of each other in the complex molecule. In some embodiments, this predetermined distance is 4.5 Angstroms. In some embodiments, this predetermined distance is 4.0 Angstroms. In some embodiments, this predetermined distance is 3.5 Angstroms.

In some embodiments, the physical property represented by $s_{i,j}$ for the corresponding pair of particles ($p_i$, $p_j$) in $\{p_1, ..., p_N\}$ is a $\pi$-$\pi$ interaction or a $\pi$-cation interaction between a first portion of the plurality of atoms represented by particle $p_i$ and a second portion of the plurality of atoms represented by particle $p_j$. A $\pi$-$\pi$ interaction is an attractive, noncovalent interaction between aromatic rings in which the aromatic rings are parallel to each other or form a T-shaped configuration and their respective centers of mass are approximately five Angstroms apart. See, for example, Brocchieri and Karlin, 1994, PNAS 91:20, 9297-9301, which is hereby incorporated by reference. A $\pi$-cation interaction is a noncovalent molecular interaction between the face of an electron-rich $\pi$ system (*e.g.* benzene, ethylene) and an adjacent cation (*e.g.* $NH_3$ group of lysine, the guanidine group of arginine, *etc.*). This interaction is an example of noncovalent bonding between a quadrupole ($\pi$ system) and a monopole (cation).

*Step 208.* Optionally, in some embodiments, a second set of physical properties $K_M$ is obtained. Whereas the physical properties $S_M$ are for pairs of particles ($p_i$, $p_j$) in $\{p_1, ..., p_N\}$, each physical property $k_i$ in $K_M$ represents a physical property of a single particle $p_i$ in $\{p_1, ..., p_N\}$. Two examples of physical properties for $K_M$ are accessible surface area and solvent-excluded surface of the plurality of atoms in the complex molecule that are represented by the corresponding particle $p_i$.

The accessible surface area (ASA), also known as the "accessible surface", is the surface area of a biomolecule that is accessible to a solvent. Measurement of ASA is usually described in units of square Angstroms. ASA is described in Lee & Richards, 1971, J. Mol. Biol. 55(3), 379-400, which is hereby incorporated by reference herein in its entirety. ASA can be calculated, for example, using the "rolling ball" algorithm developed by Shrake & Rupley, 1973, J. Mol. Biol. 79(2): 351-371, which is hereby incorporated by reference herein in its entirety. This algorithm uses a sphere (of solvent) of a particular radius to "probe" the surface of the molecule.

The solvent-excluded surface, also known as the molecular surface or Connolly surface, can be viewed as a cavity in bulk solvent (effectively the inverse of the solvent-accessible surface). It can be calculated in practice via a rolling-ball algorithm developed by Richards, 1977, Annu Rev Biophys Bioeng 6, 151-176 and implemented three-dimensionally by Connolly, 1992, J Mol Graphics 11(2), 139-141, each of which is hereby incorporated by reference herein in its entirety.

Additional examples of physical properties for $K_M$ include, but are not limited to, electrical charge, hydrophobicity, hydrophilicity, polarity, aromaticity, molecular weight and volume of the plurality of atoms in the complex molecule that are represented by the corresponding particle $p_i$.

*Step 210.* In step 210, the refined two-dimensional coordinates ($c_1$, ..., $c_N$) are plotted as a plurality of nodes 64 of a two-dimensional graph 62 after the exit condition 58 is achieved. In some embodiments, the refined two-dimensional coordinates ($c_1$, ..., $c_N$) comprises twenty-five or more nodes and step 210 comprises plotting each of these nodes 64 onto a two-dimensional graph 62. This graph can be stored in memory 36, displayed on display 32, or sent to some other output device such as a printer.

In some embodiments, after the refined two-dimensional coordinates ($c_1$, ..., $c_N$) are plotted as a plurality of nodes 64 of a two-dimensional graph 62, interaction adjustment module 72 allows for a user to adjust the position of the nodes. In this process, a user adjusts (moves) the coordinates of one or more of the nodes in the plurality of nodes as they are displayed. In some embodiments this is done by a drag and drop operation. Such manual adjustments are then saved to an updated refined set of two-dimensional coordinates ($c_1$, ..., $c_N$). This useful feature allows for the selective overriding of the cost function minimization for select nodes. The feature provides for the ability to improve the clarity of those instances where the disclosed projection onto a two dimensional plane has produced regions that are not clear. Such regions may arise, for example, when the corresponding local three dimensional structure is intrinsically complicated. In some embodiments, interaction adjustment module 72 allows for a user to delete identified nodes from the two-dimensional graph 62 in order to simplify it.

Optionally, a characteristic 66 of a node 64 in the plurality of nodes in the graph 62 is determined by a value of or a type of the physical property of the corresponding particle $p_i$ in $K_M$ 52. In some embodiments, for each respective node 64 in the plurality of nodes in the graph 62, a characteristic 66 of the respective node 64 is determined by a value of or a type of the physical property of the corresponding particle $p_i$ in $K_M$ 52. In some embodiments, the physical property $k_i$, is an accessible surface area or solvent-excluded surface of the plurality of atoms in the complex molecule that are represented by the corresponding particle $p_i$. In some embodiments, the physical property is an electrical charge, hydrophobicity, hydrophilicity, polarity, aromaticity, molecular weight or volume of the plurality of atoms in the complex molecule that are represented by the corresponding particle $p_i$.

In some embodiments, the characteristic of the node is size and a size of the respective node 64 is determined by a value of or a type of the physical property of the corresponding particle $p_i$ in $K_M$. In some embodiments, the characteristic is shading and a brightness of the shading of the respective node 64 is determined by a value of or the type of the physical property of the corresponding particle $p_i$ in $K_M$. In some embodiments, the characteristic is color and a color of the respective node 64 is determined by a value of or the type of the physical property of the corresponding particle $p_i$ in $K_m$.

In some embodiments, respective characteristics in a plurality of characteristics of the node (*e.g.*, size, shape, shading, color, *etc.*) each independently represent corresponding physical properties in a plurality of physical properties of the corresponding portion of the complex molecule represented by the corresponding particle $p_i$ in $\{p_1, ..., p_N\}$, For example, in some embodiments, one characteristic of the node is size and a size of the respective node 64 is determined by a value of or a type of a first physical property of the corresponding particle $p_i$ in $K_M$ (e.g., polarity), another characteristic is shading and a brightness of the shading of the respective node 64 is determined by a value of or the type of a second physical property of the corresponding particle $p_i$ in $K_M$ (*e.g.*, volume), and a third characteristic is color and a color of the respective node 64 is determined by a value of or the type of a third physical property of the corresponding particle $p_i$ in $K_M$ (*e.g.*, mass).

*Step 212.* In step 212, a plurality of edges 68 is plotted for the two-dimensional graph 62. Each respective edge 68 in the plurality of edges connects a two-dimensional coordinate pair $(c_i, c_j)$ (node 64) in the graph 62 that corresponds to a pair of particles $(p_i, p_j)$ in $\{p_1, ..., p_N\}$. A characteristic 70 of each respective edge 68 in the plurality of edges 68 is determined by a physical property $s_{i,j}$ in $S_M$ 50 for the pair of particles $(p_i, p_j)$ in $\{p_1, ..., p_N\}$ corresponding to the two-dimensional coordinate pair $(c_i, c_j)$ that is connected by the respective edge 68.

In some embodiments, the physical property represented by $s_{i,j}$ for the pair of particles $(p_i, p_j)$ in $\{p_1, ..., p_N\}$ is a presence of a covalent bond or hydrogen bond between a first atom in the plurality of atoms represented by particle $p_i$ and a second atom in the plurality of atoms represented by particle $p_j$. In some embodiments, the physical property represented by $s_{i,j}$ for the pair of particles $(p_i, p_j)$ in $\{p_1, ..., p_N\}$ is a presence of a carbon-carbon contact, a carbon-sulfur contact, a sulfur-sulfur contact, a carbon-nitrogen contact, or a carbon-oxygen contact between a first atom in the plurality of atoms represented by particle $p_i$ and a second atom in the plurality of atoms represented by particle $p_j$. In some embodiments, the physical property represented by $s_{i,j}$ for the pair of particles $(p_i, p_j)$ in $\{p_1, ..., p_N\}$ is a presence of a $\pi$-$\pi$ interaction or a $\pi$-cation interaction between a first atom in the plurality of atoms represented by particle $p_i$ and a second atom in the plurality of atoms represented by particle $p_j$.

In some embodiments, the characteristic is line thickness and a line thickness of an edge in the plurality of edges in the graph is determined by a value of or a type of the physical property in $S_M$ for the pair of particles $(p_i, p_j)$ in $\{p_1, ..., p_N\}$ corresponding to the two-dimensional coordinate pair $(c_i, c_j)$ that is connected by the edge. In some embodiments, the characteristic is line coloring and a color of an edge in the plurality of edges in the graph is determined by a value of or a type of the physical property in $S_M$ for the pair of particles $(p_i, p_j)$ in $\{p_1, ..., p_N\}$ corresponding to the two-dimensional coordinate pair $(c_i, c_j)$ that is connected by the edge. In some embodiments, the characteristic is line patterning and a pattern of an edge in the plurality of edges in the graph is determined by a value of or a type of the physical property in $S_M$ for the pair of particles $(p_i, p_j)$ in $\{p_1, ..., p_N\}$ corresponding to the two-dimensional coordinate pair $(c_i, c_j)$ that is connected by the edge.

In some embodiments, each characteristic in a plurality of characteristics of each respective edge 68 in the plurality of edges 68 is determined by a different physical property $s_{i,j}$ in $S_M$ 50 for the pair of particles $(p_i, p_j)$ in $\{p_1, ..., p_N\}$ corresponding to the two-dimensional coordinate pair $(c_i, c_j)$ that is connected by the respective edge 68. For example, in one such embodiment, a first characteristic in the plurality of characteristics for a respective edge 68 is line thickness and a line thickness of the edge 68 is determined by a value of or a type of a first physical property in $S_M$ for the pair of particles $(p_i, p_j)$ in $\{p_1, ..., p_N\}$ corresponding to the two-dimensional coordinate pair $(c_i, c_j)$ that is connected by the respective edge 68, a second characteristic in the plurality of characteristics for the respective edge 68 is line coloring and a color of the respective edge is determined by a value of or a type of a second physical property in $S_M$ for the pair of particles $(p_i, p_j)$ in $\{p_1, ..., p_N\}$ corresponding to the two-dimensional coordinate pair $(c_i, c_j)$ that is connected by the respective edge 68, and a third characteristic in the plurality of characteristics for the respective edge is line patterning and a pattern of the respective edge 68 is determined by a value of or a type of a third physical property in $S_M$ for the pair of particles $(p_i, p_j)$ in $\{p_1, ..., p_N\}$ corresponding to the two-dimensional coordinate pair $(c_i, c_j)$ that is connected by the respective edge 68.

In some embodiments, after the plurality of edges 68 is plotted for the two-dimensional graph 62, interaction adjustment module 72 allows for a user to adjust the position of nodes in the graph. In such embodiments, edges affected by such spatial node adjustments are automatically redrawn so that they continue to connect the same node pairs. In some embodiments, interaction adjustment module 72 allows for a user to adjust edges. In some such embodiments this is done by a drag and drop operation. In some such embodiments, nodes affected by such spatial edge adjustments are automatically repositioned so that they continue to joined by the same edges. Such manual adjustments are then saved to an updated refined set of two-dimensional coordinates $(c_1, ..., c_N)$. As in the optional embodiments described above in step 210, this useful feature allows for the selective overriding of the cost function minimization for select nodes in regions that are not clear. In some embodiments, interaction adjustment module 72 allows for a user to delete identified nodes and/or edges from the two-dimensional graph in order to simplify it.

In some embodiments, the two-dimensional graph serves as a graphical table of contents for the information pertaining to individual residues, groups of residues and/or interactions between residues of the complex molecule. In such embodiments, one or more of the nodes 64 and/or edges 68 serve as hyperlinks to free-form text or annotation. Advantageously, this simplifies the browsing and knowledge management of potentially large amount of data and information associated with the complex molecule. Thus, for example, when the two-dimensional graph 62 is shown on display 26, a user clicks on a node 64 or an edge 68 of the graph 62 thereby retrieving hyperlinked information associated with the node or edge. Typically, such hyperlinked information is for the particles $p_i$ in $\{p_1, ..., p_N\}$ corresponding to the selected node 64 or edge 68. In some embodiments, the two-dimensional graph is displayed in a web browser and, when the user clicks on a node 64 or an edge 68 of the graph 62, the hyperlinked information associated with the selected node or edge is displayed in a new browser window or in the same browser window displaying the graph 62. Such hyperlinked information can be, for example, any physical properties in $S_M$ or $K_M$, annotation information, inhibitor information (*e.g.*, binding constants, *etc*.).

*Examples.* Now that exemplary systems and methods in accordance with embodiments of the present disclosure have been presented, illustrations of the results of the systems and methods are provided. Figure 3 illustrates a three dimensional representation of the Rab4 binding domain (PDB accession code 1YZM) consisting of two slightly tilted helices in contact, in accordance with the prior art. Figure 4 illustrates the Rab4 binding domain of Figure 3 rendered as a two dimensional graph with nodes 64 (circles) and edges 68 (lines) and conveying physical information about residues of the Rab4 binding domain in accordance with the systems and methods of the present disclosure. In figure 4, solid lines connect residues that share a covalent peptide bond, thick dashed lines 402 represent hydrogen bonds where at least one of the corresponding residue partners include a side-chain atom on the hydrogen bond, dashed lines represent carbon-carbon contacts, dark gray circles represent aliphatic residues, light gray circles 404 represent aromatic residues, and white circles represent polar residues.

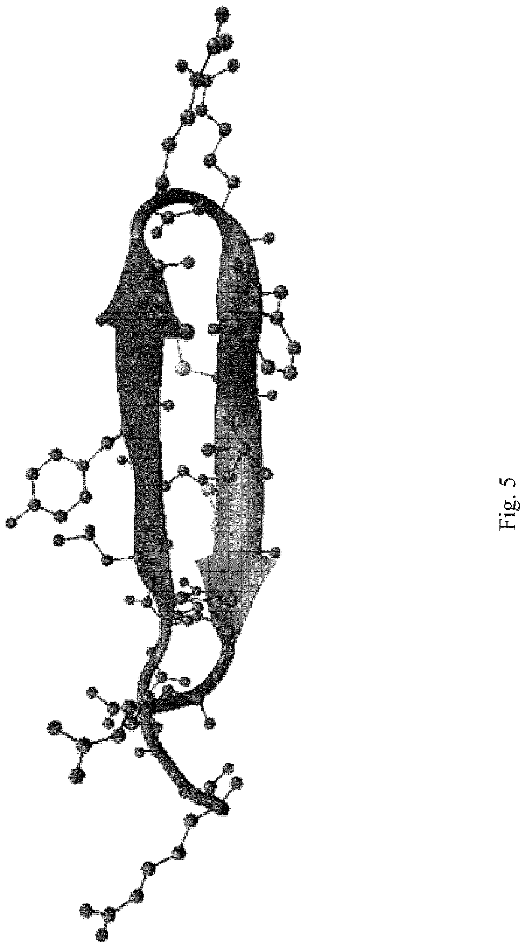

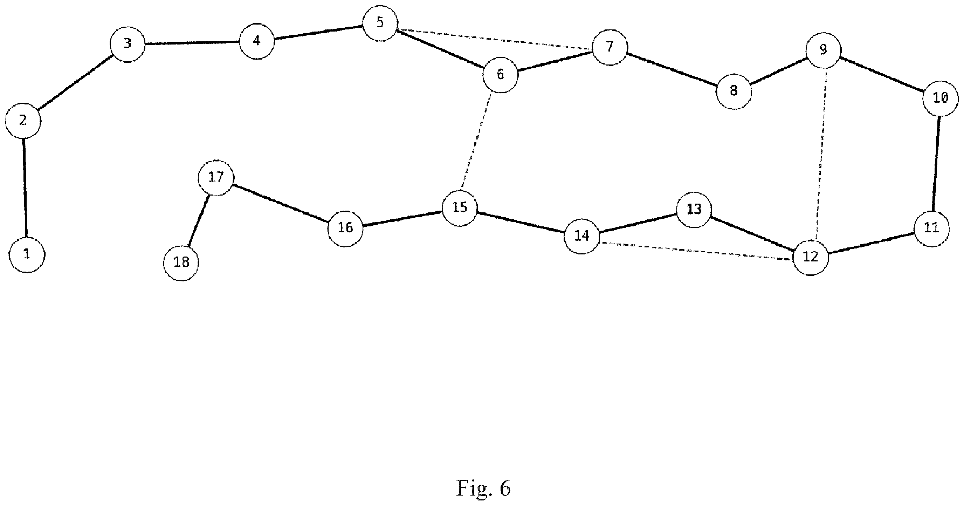

Figure 5 illustrates a three dimensional representation of the beta strand in accordance with the prior art. Figure 6 illustrates the beta strand of figure 5 rendered as a two dimensional graph with nodes 65 (circles) and edges 68 (lines) conveying physical information about residues of the beta strand of figure 5, in accordance with the systems and methods of the present disclosure.

The methods illustrated in Figure 2 may be governed by instructions that are stored in a computer readable storage medium and that are executed by at least one processor of at least one server. Each of the operations shown in Figure 2 may correspond to instructions stored in a non-transitory computer memory or computer readable storage medium. In various implementations, the non-transitory computer readable storage medium includes a magnetic or optical disk storage device, solid state storage devices such as Flash memory, or other non-volatile memory device or devices. The computer readable instructions stored on the non-transitory computer readable storage medium may be in source code, assembly language code, object code, or other instruction format that is interpreted and/or executable by one or more processors.

Plural instances may be provided for components, operations or structures described herein as a single instance. Finally, boundaries between various components, operations, and data stores are somewhat arbitrary, and particular operations are illustrated in the context of specific illustrative configurations. Other allocations of functionality are envisioned and may fall within the scope of the implementation(s). In general, structures and functionality presented as separate components in the exemplary configurations may be implemented as a combined structure or component. Similarly, structures and functionality presented as a single component may be implemented as separate components. These and other variations, modifications, additions, and improvements fall within the scope of the implementation(s).

It will also be understood that, although the terms "first," "second," etc. may be used herein to describe various elements, these elements should not be limited by these terms. These terms are only used to distinguish one element from another. For example, a first contact could be termed a second contact, and, similarly, a second contact could be termed a first contact, which changing the meaning of the description, so long as all occurrences of the "first contact" are renamed consistently and all occurrences of the second contact are renamed consistently. The first contact and the second contact are both contacts, but they are not the same contact.

As used in the description of the implementations and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if' may be construed to mean "when" or "upon" or "in response to determining" or "in accordance with a determination" or "in response to detecting," that a stated condition precedent is true, depending on the context. Similarly, the phrase "if it is determined (that a stated condition precedent is true)" or "if (a stated condition precedent is true)" or "when (a stated condition precedent is true)" may be construed to mean "upon determining" or "in response to determining" or "in accordance with a determination" or "upon detecting" or "in response to detecting" that the stated condition precedent is true, depending on the context.

The foregoing description included example systems, methods, techniques, instruction sequences, and computing machine program products that embody illustrative implementations. For purposes of explanation, numerous specific details were set forth in order to provide an understanding of various implementations of the inventive subject matter. It will be evident, however, to those skilled in the art that implementations of the inventive subject matter may be practiced without these specific details. In general, well-known instruction instances, protocols, structures and techniques have not been shown in detail.

The foregoing description, for purpose of explanation, has been described with reference to specific implementations. The implementations were chosen and described in order to best explain the principles and their practical applications, to thereby enable others skilled in the art to best utilize the implementations and various implementations with various modifications as are suited to the particular use contemplated.

**Claims**

1. A computer-implemented method for visualizing physical properties of a macromolecule in two dimensions based on three-dimensional data of the macromolecule data (44),
   wherein the macromolecule data (44) comprises the set of $\{p_1, ..., p_N\}$ particles (46), each respective particle $p_i$ in the set of particles (46) representing a different plurality of covalently bound atoms in the macromolecule, the method performed on a computer system having at least one processor and memory storing at least one program for execution by the at least one processor to perform the method, comprising:

   (A) obtaining a set of $N$ three-dimensional coordinates $\{x_1, ..., x_N\}$ (48) by x-ray crystallography, nuclear magnetic resonance spectroscopic techniques, electron microscopy, modeling or from a non-transitory computer readable storage medium, wherein each respective $x_i$ in $\{x_1, ..., x_N\}$ (48) corresponds to a particle $p_i$ in the set of $\{p_1, ..., p_N\}$ particles (46) and represents the position of $p_i$ in three-dimensional space;
   (B) minimizing a cost function (56) containing the error in a set of two-dimensional coordinates corresponding to the set of $N$ three-dimensional coordinates using a minimization function module (54):

$$E(c_1, c_2, ..., c_N) = \sum_{i<j}^{N} w_{ij} \left| \delta_{ij} - D(c_i, c_j) \right|^2$$

   using the set of $N$ three-dimensional coordinates wherein,
   $i$ and $j$ are integers greater than zero,
   $\delta_{ij}$ corresponds to a distance between a pair of three-dimensional coordinates $x_i$ and $x_j$ in $\{x_1, ..., x_N\}$ (48),
   $E(c_1, c_2, ...c_N)$ corresponds to the error in the set of two-dimensional coordinates $(c_1, ... c_N)$ wherein each two-dimensional coordinate $c_i$ in $(c_1, ... c_N)$ uniquely corresponds to a three-dimensional coordinate $x_i$ in $\{x_1, ..., x_N\}$ (48) so that each respective particle $p_i$ in the set of $\{p_1, ..., p_N\}$ particles (46) is represented by a three-dimensional coordinate $x_i$ in $\{x_1, ..., x_N\}$ (48), and a corresponding two-dimensional coordinate $c_i$ in the set of two-dimensional coordinates $(c_1, ...c_N)$,
   $D(c_i, c_j)$ corresponds to a distance between the two-dimensional coordinates $c_i$ and $c_j$ in $(c_1, ...c_N)$, and
   $w_{ij}$ is a weight for the two-dimensional pair $(p_i, p_j)$ in a matrix of weights, wherein the matrix of weights has a weight for each two-dimensional pair $(p_i, p_j)$ in the set of $\{p_1, ..., p_N\}$ particles (46),
   wherein the minimizing of the cost function refines the values of coordinates of the set of two-dimensional coordinates $(c_1, ... c_N)$ using a refinement algorithm until an exit condition (58) is achieved;

   (C) obtaining a first set of physical properties $S_M$ (50) from a non-transitory computer readable storage medium (36), each respective physical property $S_{i,j}$ in $S_M$ representing a physical property shared by a pair of particles $(p_i, p_j)$ in the set of $\{p_1, ..., p_N\}$ particles (46) in the macromolecule;
   (D) plotting the two-dimensional coordinates $(c_1, ... c_N)$, after the exit condition (58) is achieved, as a plurality of nodes (64) of a two-dimensional graph (62), wherein each node represents a portion of the macromolecule (44); and
   (E) plotting a plurality of edges (68) for the two-dimensional graph (62), wherein each respective edge (68) in the plurality of edges (68) connects a two-dimensional coordinate pair ($c_i$, $c_j$) in the two-dimensional graph (62) that corresponds to a pair of ($p_i$, $p_j$) in a set of $\{p_1, ..., p_N\}$ particles (46), and
a first characteristic of each respective edge (68) in the plurality of edges (68) is determined by a value of or a type of physical property $S_{i,j}$ in $S_M$ for the pair of particles ($p_i$, $p_j$) in the set of $\{p_1, ..., p_N\}$ particles (46) corresponding to the two-dimensional coordinate pair ($c_i$, $c_j$) that is connected by the respective edge (68).

2. The computer-implemented visualization method of claim 1, wherein the weights are defined as:

$$w_{ij} = \frac{1}{\delta_{ij}} \frac{1}{\sum_{k<l}^{N} \delta_{kl}}$$

wherein,
$\delta_{kl}$ corresponds to a distance between a pair of three-dimensional coordinates $x_k$ and $x_l$ in $\{x_1, ..., x_N\}$ (48).

3. The computer-implemented visualization method of claim 2, wherein the refinement algorithm is steepest descent in which the derivative of the cost function is expressed as:

$$= \frac{-2}{\sum_{k<l}^{N} \delta_{kl}} \sum_{j,j \neq m}^{N} \frac{1}{\delta_{mj}} \left| \delta_{mj} - D(c_m, c_j) \right| \frac{(c_m - c_j)}{D(c_m, c_j)}.$$

wherein, $j$, $k$, $l$ and $m$ are integers greater than zero,
$\delta_{mj}$ corresponds to a distance between a pair of three-dimensional coordinates $x_m$ and $xj$ in $\{x_1, ..., x_N\}$ (48),
$D(c_m, c_j)$ corresponds to a distance between the two-dimensional coordinates $c_m$ and $c_j$ in ($c_1, ... c_N$), and
$\delta_{k,l}$ corresponds to a distance between a pair of three-dimensional coordinates $x_k$ and $x_l$ in $\{x_1, ..., x_N\}$ (48).

4. The computer-implemented visualization method of any one of claims 1 through 3, wherein the macromolecule is a polypeptide,

each particle $p_i$ in the set of $\{p_1, ..., p_N\}$ particles (46) represents a residue in the polypeptide, and
each respective $x_i$ in $\{x_1, ..., x_N\}$ (48) corresponds to the three-dimensional coordinates of the $C_\alpha$ carbon of each respective residue represented by the $p_i$ in the set of $\{p_1, ..., p_N\}$ particles (46) that corresponds to the respective $x_i$.

5. The computer-implemented visualization method of any one of claims 1 through 3, wherein the macromolecule is a polynucleic acid, a polyribonucleic acid, a polysaccharide, or a polypeptide.

6. The computer-implemented visualization method of any one of claims 1 through 3, wherein

(i) the macromolecule is a polynucleic acid and each respective particle $p_i$ in the set of $\{p_1, ..., p_N\}$ particles (46) represents a nucleic acid residue in the polynucleic acid, or
(ii) the macromolecule is a polyribonucleic acid and each respective particle $p_i$ in the set of $\{p_1, ..., p_N\}$ particles (46) represents a ribonucleic acid residue in the polyribonucleic acid, or
(iii) the macromolecule is a polysaccharide and each respective particle $p_i$ in the set of $\{p_1, ..., p_N\}$ particles (46) represents a monosaccharide unit or a disaccharide unit in the polysaccharide, or
(iv) the macromolecule is a polypeptide and each respective particle $p_i$ in the set of $\{p_1, ..., p_N\}$ particles (46) represents a residue in the polypeptide, or
(v) the macromolecule is a surfactant, organometallic compound, fullerene, or polymer.

7. The computer-implemented visualization method of any one of claims 1 through 6, wherein the physical property represented by $S_{i,j}$ shared by each respective pair of particles ($p_i$, $p_j$) in a set of $\{p_1, ..., p_N\}$ particles (46) in the macromolecule, is

(i) a presence of a covalent bond between a first atom in the plurality of atoms represented by particle $p_i$ and a second atom in the plurality of atoms represented by particle $p_j$, or
(ii) a presence of a hydrogen bond between a first atom in the plurality of atoms represented by particle $p_i$ and a second atom in the plurality of atoms represented by particle $p_j$, or
(iii) a presence of a carbon-carbon contact, a carbon-sulfur contact, a sulfur-sulfur contact, a carbon-nitrogen contact, or a carbon-oxygen contact between a first atom in the plurality of atoms represented by particle $p_i$ and a second atom in the plurality of atoms represented by particle $p_j$ or

(iv) a $\pi$-$\pi$ interaction or a $\pi$-cation interaction between a first portion of the plurality of atoms represented by particle $p_i$ and a second atom in the plurality of atoms represented by particle $p_j$.

**8.** The computer-implemented visualization method of any one of claims 1 through 7, wherein the first characteristic of each respective edge in step (E) is

(i) line thickness and a line thickness of an edge in the plurality of edges in the two-dimensional graph (62) is determined by a value of or a type of the physical property in $S_M$ for each respective pair of particles ($p_i$, $p_j$) in the set of $\{p_1, ..., p_N\}$ particles (46) corresponding to each respective two-dimensional coordinate pair ($c_i$, $c_j$) that is connected by the edge, or

(ii) line coloring and a color of an edge in the plurality of edges in the two-dimensional graph (62) is determined by a value of or a type of the physical property in $S_M$ for each respective pair of particles ($p_i$, $p_j$) in the set of $\{p_1, ..., p_N\}$ particles (46) corresponding to each respective two-dimensional coordinate pair ($c_i$, $c_j$) that is connected by the edge, or

(iii) line patterning and a pattern of an edge in the plurality of edges in the two-dimensional graph (62) is determined by a value of or a type of the physical property in $S_M$ for each respective pair of particles ($p_i$, $p_j$) in the set of $\{p_1, ..., p_N\}$ particles (46) corresponding to each respective two-dimensional coordinate pair ($c_i$, $c_j$) that is connected by the edge.

**9.** The computer-implemented visualization method of any one of claims 1 through 8, the method further comprising: obtaining a second set of physical properties $K_M$ (52), each physical property $k_i$ in $K_M$ (52) representing a physical property of a corresponding particle $p_i$ in the set of $\{p_1, ..., p_N\}$ particles (46), and wherein a second characteristic of each respective node (64) in the plurality of nodes (64) in the two-dimensional graph (62) is determined by a value of or a type of the physical property of the corresponding particle $p_i$ in $K_M$ (52).

**10.** The computer-implemented visualization method of claim 9, wherein the physical property $k_i$ corresponds to

(i) an accessible surface area or solvent-excluded surface of the plurality of atoms in the macromolecule that are represented by the corresponding particle $p_i$, or

(ii) an electrical charge, hydrophobicity, hydrophilicity, polarity, aromaticity, molecular weight or volume of the plurality of atoms in the macromolecule that are represented by the corresponding particle $p_i$.

**11.** The computer-implemented visualization method of claim 9, wherein the second characteristic is

(i) shading and a brightness of the respective node (64) is determined by a value of or a type of the physical property of the corresponding particle $p_i$ in $K_M$ (52), or

(ii) size and a size of the respective node (64) is determined by a value of or a type of the physical property of the corresponding particle $p_i$ in $K_M$ (52), or

(iii) color and a color of the respective node (64) is determined by a value of or a type of the physical property of the corresponding particle $p_i$ in $K_M$ (52).

**12.** The computer-implemented visualization method of any one of claims 1 through 11, wherein the exit condition is achieved when a predetermined maximum number of iterations of the refinement algorithm have been computed.

**13.** A computer system configured for visualizing physical properties of a macromolecule in two dimensions based on three-dimensional data of the macromolecule data (44), wherein the macromolecule data (44) comprises the set of $\{p_1, ..., p_N\}$ particles (46), each respective particle $p_i$ in the set of particles (46) representing a different plurality of covalently bound atoms in the macromolecule, the computer system comprising:

at least one processor; and
memory storing at least one computer program,

wherein the at least one processor is configured to execute the at least one program comprising instructions for:

(A) obtaining a set of $N$ three-dimensional coordinates $\{x_1, ..., x_N\}$ (48) by x-ray crystallography, nuclear magnetic resonance spectroscopic techniques, electron microscopy, modeling or from a non-transitory computer readable storage medium, wherein each respective $x_i$ in $\{x_1, ..., x_N\}$ (48) corresponds to a particle $p_i$ in the set of $\{p_1, ..., p_N\}$ particles (46) and represents the position of $p_i$ in three-dimensional space;

(B) minimizing a cost function (56) containing the error in a set of two-dimensional coordinates corresponding to the set of $N$ three-dimensional coordinates using a minimization function module (54):

$$E(c_1, c_2, ..., c_N) = \sum_{i<j}^{N} w_{ij} |\delta_{ij} - D(c_i, c_j)|^2$$

using the set of $N$ three-dimensional coordinates wherein,

$i$ and $j$ are integers greater than zero,

$\delta_{ij}$ corresponds to a distance between a pair of three-dimensional coordinates $x_i$ and $x_j$ in $\{x_1, ..., x_N\}$ (48),

$E(c_1, c_2, ...c_N)$ corresponds to the error in the set of two-dimensional coordinates $(c_1, ... c_N)$ wherein each two-dimensional coordinate $c_i$ in $(c_1, ... c_N)$ uniquely corresponds to a three-dimensional coordinate $x_i$ in $\{x_1, ..., x_N\}$ (48) so that each respective particle $p_i$ in the set of $\{p_1, ..., p_N\}$ particles (46) is represented by a three-dimensional coordinate $x_i$ in $\{x_1, ..., x_N\}$ (48), and a corresponding two-dimensional coordinate $c_i$ in the set of two-dimensional coordinates $(c_1, ...c_N)$,

$D(c_i, c_j)$ corresponds to a distance between the two-dimensional coordinates $c_i$ and $c_j$ in $(c_1, ...c_N)$, and

$w_{ij}$ is a weight for the two-dimensional pair $(p_i, p_j)$ in a matrix of weights, wherein the matrix of weights has a weight for each two-dimensional pair $(p_i, p_j)$ in the set of $\{p_1, ..., p_N\}$ particles (46),

wherein the minimizing of the cost function refines the values of coordinates of the set of two-dimensional coordinates $(c_1, ... c_N)$ using a refinement algorithm until an exit condition (58) is achieved;

(C) obtaining a first set of physical properties $S_M$ (50) from a non-transitory computer readable storage medium (36), each respective physical property $S_{i,j}$ in $S_M$ representing a physical property shared by a pair of particles $(p_i, p_j)$ in the set of $\{p_1, ..., p_N\}$ particles (46) in the macromolecule;

(D) plotting the two-dimensional coordinates $(c_1, ... c_N)$, after the exit condition (58) is achieved, as a plurality of nodes (64) of a two-dimensional graph (62), wherein each node represents a portion of the macromolecule (44);

(E) plotting a plurality of edges (68) for the two-dimensional graph (62), wherein

each respective edge (68) in the plurality of edges (68) connects a two-dimensional coordinate pair $(c_i, c_j)$ in the two-dimensional graph (62) that corresponds to a pair of $(p_i, p_j)$ in a set of $\{p_1, ..., p_N\}$ particles (46), and a first characteristic of each respective edge (68) in the plurality of edges (68) is determined by a value of or a type of physical property $S_{i,j}$ in $S_M$ for the pair of particles $(p_i, p_j)$ in the set of $\{p_1, ..., p_N\}$ particles (46) corresponding to the two-dimensional coordinate pair $(c_i, c_j)$ that is connected by the respective edge (68).

14. A non-transitory computer readable storage medium adapted to store a visualization module for visualizing physical properties of a macromolecule in two dimensions based on three-dimensional data of the macromolecule data (44), wherein the macromolecule data (44) comprises the set of $\{p_1, ..., p_N\}$ particles (46), each respective particle $p_i$ in the set of particles (46) representing a different plurality of covalently bound atoms in the macromolecule, wherein the visualization module comprises instructions which when executed on a computer performs:

(A) obtaining a set of $N$ three-dimensional coordinates $\{x_1, ..., x_N\}$ (48) by x-ray crystallography, nuclear magnetic resonance spectroscopic techniques, electron microscopy, modeling or from a non-transitory computer readable storage medium, wherein each respective $x_i$ in $\{x_1, ..., x_N\}$ (48) corresponds to a particle $p_i$ in the set of $\{p_1, ..., p_N\}$ particles (46) and represents the position of $p_i$ in three-dimensional space;

(B) minimizing a cost function (56) containing the error in a set of two-dimensional coordinates corresponding to the set of $N$ three-dimensional coordinates using a minimization function module (54):

$$E(c_1, c_2, ..., c_N) = \sum_{i<j}^{N} w_{ij} |\delta_{ij} - D(c_i, c_j)|^2$$

using the set of $N$ three-dimensional coordinates wherein,

$i$ and $j$ are integers greater than zero,

$\delta_{ij}$ corresponds to a distance between a pair of three-dimensional coordinates $x_i$ and $x_j$ in $\{x_1, ..., x_N\}$ (48),

$E(c_1, c_2, ...c_N)$ corresponds to the error in the set of two-dimensional coordinates $(c_1, ... c_N)$ wherein each

two-dimensional coordinate $c_i$ in $(c_1, ... c_N)$ uniquely corresponds to a three-dimensional coordinate $x_i$ in $\{x_1, ..., x_N\}$ (48) so that each respective particle $p_i$ in the set of $\{p_1, ..., p_N\}$ particles (46) is represented by a three-dimensional coordinate $x_i$ in $\{x_1, ..., x_N\}$ (48), and a corresponding two-dimensional coordinate $c_i$ in the set of two-dimensional coordinates $(c_1, ...c_N)$,

$D(c_i, c_j)$ corresponds to a distance between the two-dimensional coordinates $c_i$ and $c_j$ in $(c_1, ...c_N)$, and $w_{i,j}$ is a weight for the two-dimensional pair $(p_i, p_j)$ in a matrix of weights, wherein the matrix of weights has a weight for each two-dimensional pair $(p_i, p_j)$ in the set of $\{p_1, ..., p_N\}$ particles (46),

wherein the minimizing of the cost function refines the values of coordinates of the set of two-dimensional coordinates $(c_1, ... c_N)$ using a refinement algorithm until an exit condition (58) is achieved;

(C) obtaining a first set of physical properties $S_M$ (50) from a non-transitory computer readable storage medium (36), each respective physical property $S_{i,j}$ in $S_M$ representing a physical property shared by a pair of particles $(p_i, p_j)$ in the set of $\{p_1, ..., p_N\}$ particles (46) in the macromolecule;

(D) plotting the two-dimensional coordinates $(c_1, ... c_N)$, after the exit condition (58) is achieved, as a plurality of nodes (64) of a two-dimensional graph (62), wherein each node represents a portion of the macromolecule (44);

(E) plotting a plurality of edges (68) for the two-dimensional graph (62), wherein each respective edge (68) in the plurality of edges (68) connects a two-dimensional coordinate pair $(c_i, c_j)$ in the two-dimensional graph (62) that corresponds to a pair of $(p_i, p_j)$ in a set of $\{p_1, ..., p_N\}$ particles (46), and

a first characteristic of each respective edge (68) in the plurality of edges (68) is determined by a value of or a type of physical property $S_{i,j}$ in $S_M$ for the pair of particles $(p_i, p_j)$ in the set of $\{p_1, ..., p_N\}$ particles (46) corresponding to the two-dimensional coordinate pair $(c_i, c_j)$ that is connected by the respective edge (68).

**15.** The computer-implemented visualization method of claim 1, wherein

(i) the plotting step (D) further comprises accepting a user-inputted manual spatial change to a two-dimensional coordinate $c_i$ in $(c_1, ...c_N)$, or

(ii) the plotting step (D) further comprises accepting a user-inputted deletion of a two-dimensional coordinate $c_i$ in $(c_1, ...c_N)$, or

(iii) the plotting step (E) further comprises accepting a user-inputted manual spatial change of a two-dimensional coordinate $c_i$ in $(c_1, ...c_N)$ or an edge (68) in the plurality of edges (68), or

(iv) the plotting step (E) further comprises accepting a user-inputted manual spatial change of a two-dimensional coordinate $c_i$ in $(c_1, ...c_N)$ and, responsive to accepting the manual spatial change to the $c_i$, updating an edge (68) associated with the $c_i$, or

(v) the plotting step (E) further comprises accepting a user-inputted manual spatial change to an edge (68) in the plurality of edges (68) and, responsive to accepting the manual spatial change to the edge (68), updating a $c_i$ in $(c_1, ...c_N)$ associated with the $c_i$ without user intervention, or

(vi) the plotting step (E) further comprises accepting a user-inputted deletion of a two-dimensional coordinate $c_i$ in $(c_1, ...c_N)$ or an edge (68) in the plurality of edges (68).

**Patentansprüche**

**1.** Ein computerimplementiertes Verfahren zur Visualisierung von physikalischen Eigenschaften eines Makromoleküls in zwei Dimensionen, basierend auf dreidimensionalen Daten der Makromoleküldaten (44),

wobei die Makromoleküldaten (44) einen Satz von $\{p_1, ..., p_N\}$ Partikeln (46) umfassen, wobei jedes jeweilige Partikel $p_i$ in dem Satz von Partikeln (46) eine unterschiedliche Vielzahl kovalent gebundener Atome in dem Makromolekül darstellt, wobei das Verfahren auf einem Computersystem durchgeführt wird, das mindestens einen Prozessor und Speicher hat, der mindestens ein Programm zur Ausführung durch den mindestens einen Prozessor speichert, um das Verfahren durchzuführen, umfassend:

(A) Erhalten einen Satzes von $N$ dreidimensionalen Koordinaten $\{x_1, ..., x_N\}$ (48) durch Röntgenkristallographie, Kernmagentresonanz-spektroskopische Techniken, Elektronenmikroskopie, Modellieren oder von einem nichtvergänglichen computerlesbaren Speichermedium, wobei jedes jeweilige $x_i$ in $\{x_1, ..., x_N\}$ (48) einem Partikel $p_i$ im Satz von $\{p_1, ..., p_N\}$ Partikeln (46) entspricht und die Position von $p_i$ im dreidimensionalen Raum darstellt;

(B) Minimieren einer Kostenfunktion (56) umfassend den Fehler in einem Satz zweidimensionaler Koordinaten, die dem Satz von $N$ dreidimensionalen Koordinaten entsprechen, unter Verwendung eines Minimierungsfunktionsmoduls (54):

$$E(c_1, c_2, \ldots, c_N) = \sum_{i<j}^{N} w_{ij} \left| \delta_{ij} - D(c_i, c_j) \right|^2$$

unter Verwendung des Satzes von *N* dreidimensionalen Koordinaten, wobei

*i* und *j* ganze Zahlen größer als Null sind,

$\delta_{ij}$ einer Entfernung zwischen einem Paar dreidimensionaler Koordinaten $x_i$ und $x_j$ in $\{x_1, \ldots, x_N\}$ (48) entspricht,

$E(c_1, c_2, \ldots c_N)$ dem Fehler in dem Satz zweidimensionaler Koordinaten $(c_1, \ldots c_N)$ entspricht, wobei jede zweidimensionale Koordinate $c_i$ in $(c_1, \ldots c_N)$ eindeutig einer dreidimensionalen Koordinate $x_i$ in $\{x_1, \ldots, x_N\}$ (48) entspricht, so dass jedes jeweilige Partikel $p_i$ im Satz von $\{p_1, \ldots, p_N\}$ Partikeln (46) durch eine dreidimensionale Koordinate $x_i$ in $\{x_1, \ldots, x_N\}$ (48) und eine entsprechende zweidimensionale Koordinate $c_i$ im Satz zweidimensionaler Koordinaten $(c_1, \ldots c_N)$ dargestellt wird,

$D(c_i, c_j)$ einer Entfernung zwischen den zweidimensionalen Koordinaten $c_i$ und $c_j$ in $(c_1, \ldots c_N)$ entspricht, und $w_{ij}$ eine Gewichtung für das zweidimensionale Paar $(p_i, p_j)$ in einer Matrix von Gewichtungen ist, wobei die Matrix von Gewichtungen eine Gewichtung für jedes zweidimensionale Paar $(p_i, p_j)$ im Satz von $\{p_1, \ldots, p_N\}$ Partikeln (46) hat,

wobei das Minimieren der Kostenfunktion die Werte der Koordinaten des Satzes zweidimensionaler Koordinaten $(c_1, \ldots c_N)$ unter Verwendung eines Verfeinerungsalgorithmus verfeinert, bis eine Austrittsbedingung (58) erreicht ist;

(C) Erhalten eines ersten Satzes physikalischer Eigenschaften $S_M$ (50) von einem nichtvergänglichen computerlesbaren Speichermedium (36), wobei jede jeweilige physikalische Eigenschaft $S_{i,j}$ in $S_M$ eine physikalische Eigenschaft darstellt, die von einem Paar von Partikeln $(p_i, p_j)$ im Satz von $\{p_1, \ldots, p_N\}$ Partikeln (46) im Makromolekül geteilt wird;

(D) Auftragen der zweidimensionalen Koordinaten $(c_1, \ldots c_N)$, nachdem die Austrittsbedingung (58) erreicht ist, als eine Vielzahl von Knoten (64) eines zweidimensionalen Graphs (62), wobei jeder Knoten einen Teil des Makromoleküls (44) darstellt; und

(E) Auftragen einer Vielzahl von Kanten (68) für den zweidimensionalen Graphen (62), wobei jede jeweilige Kante (68) in der Vielzahl von Kanten (68) ein zweidimensionales Koordinatenpaar $(c_i, c_j)$ im zweidimensionalen Graphen (62) verbindet, das einem Paar $(p_i, p_j)$ in einem Satz von $\{p_1, \ldots, p_N\}$ Partikeln (46) entspricht, und ein erstes Charakteristikum jeder jeweiligen Kante (68) in der Vielzahl von Kanten (68) wird bestimmt durch einen Wert von oder einen Typ von physikalischer Eigenschaft $S_{i,j}$ in $S_M$ für das Paar von Partikeln $(p_i, p_j)$ im Satz von $\{p_1, \ldots, p_N\}$ Partikeln (46), die dem zweidimensionalen Koordinatenpaar $(c_i, c_j)$ entsprechen, das durch die jeweilige Kante (68) verbunden ist.

2. Das computerimplementierte Visualisierungsverfahren aus Anspruch 1, wobei die Gewichtungen definiert sind als:

$$w_{ij} = \frac{1}{\delta_{ij}} \frac{1}{\sum_{k<l}^{N} \delta_{kl}}$$

wobei
$\delta_{ki}$ einer Entfernung zwischen einem Paar dreidimensionaler Koordinaten $x_k$ und $x_l$ in $\{x_1, \ldots, x_N\}$ (48) entspricht.

3. Das computerimplementierte Visualisierungsverfahren aus Anspruch 2, wobei der Verfeinerungsalgorithmus steilster Abfall ist, in dem die Ableitung der Kostenfunktion ausgedrückt wird als:

$$= \frac{-2}{\sum_{k<l}^{N} \delta_{kl}} \sum_{j, j \neq m}^{N} \frac{1}{\delta_{mj}} \left| \delta_{mj} - D(c_m, c_j) \right| \frac{(c_m - c_j)}{D(c_m, c_j)}.$$

wobei *j*, *k*, *l* und m ganze Zahlen größer Null sind,
$\delta_{mj}$ einer Entfernung zwischen einem Paar dreidimensionaler Koordinaten $x_m$ und $xj$ in $\{x_1, \ldots, x_N\}$ (48) entspricht,
$D(C_m, c_j)$ einer Entfernung zwischen den zweidimensionalen Koordinaten $c_m$ und $c_j$ in $(c_1, \ldots c_N)$ entspricht, und
$\delta_{k,l}$ einer Entfernung zwischen einem Paar dreidimensionaler Koordinaten $x_k$ und $x_l$ in $\{x_1, \ldots, x_N\}$ (48) entspricht.

**4.** Das computerimplementierte Visualisierungsverfahren aus irgendeinem der Ansprüche 1 bis 3, wobei das Makromolekül ein Polypeptid ist,

jedes Partikel $p_i$ im Satz von $\{p_1, ..., p_N\}$ Partikeln (46) einen Rest im Polypeptid darstellt, und jedes jeweilige $x_i$ in $\{x_1, ..., x_N\}$ (48) den dreidimensionalen Koordinaten des $C_\alpha$-Kohlenstoffs jedes jeweiligen durch die $p_i$ im Satz von $\{p_1, ..., p_N\}$ Partikeln (46) dargestellten Restes entspricht, das dem jeweiligen $x_i$ entspricht.

**5.** Das computerimplementierte Visualisierungsverfahren aus irgendeinem der Ansprüche 1 bis 3, wobei das Makromolekül eine Polynukleinsäure, eine Polyribonukleinsäure, ein Polysaccharid oder ein Polypeptid ist.

**6.** Das computerimplementierte Visualisierungsverfahren aus irgendeinem der Ansprüche 1 bis 3, wobei

(i) das Makromolekül eine Polynukleinsäure ist und jedes jeweilige Partikel $p_i$ im Satz von $\{p_1, ..., p_N\}$ Partikeln (46) einen Nukleinsäurerest in der Polynukleinsäure darstellt, oder
(ii) das Makromolekül eine Polyribonukleinsäure ist und jedes jeweilige Partikel $p_i$ im Satz von $\{p_1, ..., p_N\}$ Partikeln (46) einen Ribonukleinsäurerest in der Polyribonukleinsäure darstellt, oder
(iii) das Makromolekül ein Polysaccharid ist und jedes jeweilige Partikel $p_i$ im Satz von $\{p_1, ..., p_N\}$ Partikeln (46) eine Monosaccharideinheit oder eine Disaccharideinheit im Polysaccharid darstellt, oder
(iv) das Makromolekül ein Polypeptid ist und jedes jeweilige Partikel $p_i$ im Satz von $\{p_1, ..., p_N\}$ Partikeln (46) einen Rest im Polypeptid darstellt, oder
(v) das Makromolekül ein Tensid, eine organometallische Verbindung, ein Fulleren oder ein Polymer ist.

**7.** Das computerimplementierte Visualisierungsverfahren aus irgendeinem der Ansprüche 1 bis 6, wobei die durch $S_{i,j}$ dargestellte physikalische Eigenschaft, die von jedem jeweiligen Paar von Partikeln $(p_i, p_j)$ im Satz von $\{p_1, ..., p_N\}$ Partikeln (46) im Makromolekül geteilt wird,

(i) das Vorliegen einer kovalenten Bindung zwischen einem ersten Atom in der Vielzahl von Atomen, dargestellt durch Partikel $p_i$, und einem zweiten Atom in der Vielzahl von Atomen, dargestellt durch Partikel $p_j$, oder
(ii) das Vorliegen einer Wasserstoffbrückenbindung zwischen einem ersten Atom in der Vielzahl von Atomen, dargestellt durch Partikel $p_i$, und einem zweiten Atom in der Vielzahl von Atomen, dargestellt durch Partikel $p_j$, oder
(iii) das Vorliegen eines Kohlenstoff-Kohlenstoff-Kontakts, eines Kohlenstoff-Schwefel-Kontakts, eines Schwefel-Schwefel-Kontakts, eines Kohlenstoff-Stickstoff-Kontakts oder eines Kohlenstoff-Sauerstoff-Kontakts zwischen einem ersten Atom in der Vielzahl von Atomen, dargestellt durch Partikel $p_i$, und einem zweiten Atom in der Vielzahl von Atomen, dargestellt durch Partikel $p_j$, oder
(iv) eine π-π-Wechselwirkung oder eine n-Kation-Wechselwirkung zwischen einem ersten Teil der Vielzahl von Atomen, dargestellt durch Partikel $p_i$, und einem zweiten Atom in der Vielzahl von Atomen, dargestellt durch Partikel $p_j$,
ist.

**8.** Das computerimplementierte Visualisierungsverfahren aus irgendeinem der Ansprüche 1 bis 7, wobei das erste Charakteristikum jeder jeweiligen Kante in Schritt (E)

(i) Liniendicke ist, und Liniendicke einer Kante in der Vielzahl von Kanten im zweidimensionalen Graphen (62) durch einen Wert von oder einen Typ von der physikalischen Eigenschaft in $S_M$ für jedes jeweilige Paar von Partikeln $(p_i, p_j)$ im Satz von $\{p_1, ..., p_N\}$ Partikeln (46) bestimmt wird, die jedem jeweiligen zweidimensionalen Koordinatenpaar $(c_i, c_j)$ entsprechen, das durch die Kante verbunden wird, oder
(ii) Linienfärbung ist und die Farbe einer Kante in der Vielzahl von Kanten im zweidimensionalen Graphen (62) durch einen Wert von oder einen Typ von der physikalischen Eigenschaft in $S_M$ für jedes jeweilige Paar von Partikeln $(p_i, p_j)$ im Satz von $\{p_1, ..., p_N\}$ Partikeln (46) bestimmt wird, die jedem jeweiligen zweidimensionalen Koordinatenpaar $(c_i, c_j)$ entsprechen, das durch die Kante verbunden wird, oder
(iii) Linienmuster ist, und das Muster einer Kante in der Vielzahl von Kanten im zweidimensionalen Graphen (62) durch einen Wert von oder einen Typ von der physikalischen Eigenschaft in $S_M$ für jedes jeweilige Paar von Partikeln $(p_i, p_j)$ im Satz von $\{p_1, ..., p_N\}$ Partikeln (46) bestimmt wird, die jedem jeweiligen zweidimensionalen Koordinatenpaar $(c_i, c_j)$ entsprechen, das durch die Kante verbunden wird.

**9.** Das computerimplementierte Visualisierungsverfahren aus irgendeinem der Ansprüche 1 bis 8, das Verfahren weiter umfassend:

Erhalten eines zweiten Satzes physikalischer Eigenschaften $K_M$ (52), wobei jede physikalische Eigenschaft $k_i$ in $K_M$ (52) eine physikalische Eigenschaft eines entsprechenden Partikels $p_i$ im Satz von $\{p_1, ..., p_N\}$ Partikeln (46) darstellt, und wobei ein zweites Charakteristikum jedes jeweiligen Knotens (64) in der Vielzahl von Knoten (64) im zweidimensionalen Graphen (62) durch einen Wert von oder einen Typ von der physikalischen Eigenschaft des entsprechenden Partikels $p_i$ in $K_M$ (52) bestimmt wird.

10. Das computerimplementierte Visualisierungsverfahren aus Anspruch 9, wobei die physikalische Eigenschaft $k_i$

(i) einer zugänglichen Oberfläche oder Lösungsmittel-ausgeschlossenen Oberfläche der Vielzahl von Atomen im Makromolekül, die durch das entsprechende Partikel $p_i$ dargestellt werden, oder
(ii) einer elektrischen Ladung, Hydrophobie, Hydrophilie, Polarität, Aromatizität, Molekulargewicht oder Volumen der Vielzahl von Atomen im Makromolekül, die durch das entsprechende Partikel $p_i$ dargestellt werden, entspricht.

11. Das computerimplementierte Visualisierungsverfahren aus Anspruch 9, wobei das zweite Charakteristikum

(i) Schattierung ist und eine Helligkeit des jeweiligen Knotens (64) durch einen Wert von oder einen Typ von der physikalischen Eigenschaft des entsprechenden Partikels $p_i$ in $K_M$ (52) bestimmt wird, oder
(ii) Größe ist und eine Größe des jeweiligen Knotens (64) durch einen Wert von oder einen Typ von der physikalischen Eigenschaft des entsprechenden Partikels $p_i$ in $K_M$ (52) bestimmt wird, oder
(iii) Farbe ist und eine Farbe des jeweiligen Knotens (64) durch einen Wert von oder einen Typ von der physikalischen Eigenschaft des entsprechenden Partikels $p_i$ in $K_M$ (52) bestimmt wird.

12. Das computerimplementierte Visualisierungsverfahren aus irgendeinem der Ansprüche 1 bis 11, wobei die Austrittsbedingung erreicht ist, wenn eine vorbestimmte Höchstzahl von Wiederholungen des Verfeinerungsalgorithmus berechnet worden sind.

13. Ein Computersystem, konfiguriert zur Visualisierung physikalischer Eigenschaften eines Makromoleküls in zwei Dimensionen, basierend auf dreidimensionalen Daten der Makromoleküldaten (44), wobei die Makromoleküldaten (44) einen Satz von $\{p_1, ..., p_N\}$ Partikeln (46) umfassen, wobei jedes jeweilige Partikel $p_i$ in dem Satz von Partikeln (46) eine unterschiedliche Vielzahl kovalent gebundener Atome in dem Makromolekül darstellt, das Computersystem umfassend:

mindestens einen Prozessor; und
Speicher, der mindestens ein Computerprogramm speichert,

wobei der mindestens eine Prozessor konfiguriert ist, das mindestens eine Programm auszuführen, das Anweisungen umfasst für:

(A) Erhalten einen Satzes von N dreidimensionalen Koordinaten $\{x_1, ..., x_N\}$ (48) durch Röntgenkristallographie, Kernmagentresonanz-spektroskopische Techniken, Elektronenmikroskopie, Modellieren oder von einem nichtvergänglichen computerlesbaren Speichermedium, wobei jedes jeweilige $x_i$ in $\{x_1, ..., x_N\}$ (48) einem Partikel $p_i$ im Satz von $\{p_1, ..., p_N\}$ Partikeln (46) entspricht und die Position von $p_i$ im dreidimensionalen Raum darstellt;
(B) Minimieren einer Kostenfunktion (56) umfassend den Fehler in einem Satz zweidimensionaler Koordinaten, die dem Satz von N dreidimensionalen Koordinaten entsprechen, unter Verwendung eines Minimierungsfunktionsmoduls (54):

$$E(c_1, c_2, ..., c_N) = \sum_{i<j}^{N} w_{ij} \left| \delta_{ij} - D(c_i, c_j) \right|^2$$

unter Verwendung des Satzes von N dreidimensionalen Koordinaten, wobei
$i$ und $j$ ganze Zahlen größer als Null sind,
$\delta_{ij}$ einer Entfernung zwischen einem Paar dreidimensionaler Koordinaten $x_i$ und $x_j$ in $\{x_1, ..., x_N\}$ (48) entspricht,
$E(c_1, c_2,...c_N)$ dem Fehler in dem Satz zweidimensionaler Koordinaten $(c_1,...c_N)$ entspricht, wobei jede

zweidimensionale Koordinate $c_i$ in $(c_1,...c_N)$ eindeutig einer dreidimensionalen Koordinate $x_i$ in $\{x_1, ..., x_N\}$ (48) entspricht, so dass jedes jeweilige Partikel $p_i$ im Satz von $\{p_1, ..., p_N\}$ Partikeln (46) durch eine dreidimensionale Koordinate $x_i$ in $\{x_1, ..., x_N\}$ (48) und eine entsprechende zweidimensionale Koordinate $c_i$ im Satz zweidimensionaler Koordinaten $(c_1,...c_N)$ dargestellt wird,

$D(c_i, c_j)$ einer Entfernung zwischen den zweidimensionalen Koordinaten $c_i$ und $c_j$ in $(c_1,...c_N)$ entspricht, und $w_{ij}$ eine Gewichtung für das zweidimensionale Paar $(p_i, p_j)$ in einer Matrix von Gewichtungen ist, wobei die Matrix von Gewichtungen eine Gewichtung für jedes zweidimensionale Paar $(p_i, p_j)$ im Satz von $\{p_1, ..., p_N\}$ Partikeln (46) hat,

wobei das Minimieren der Kostenfunktion die Werte der Koordinaten des Satzes zweidimensionaler Koordinaten $(c_1,...c_N)$ unter Verwendung eines Verfeinerungsalgorithmus verfeinert, bis eine Austrittsbedingung (58) erreicht ist;

(C) Erhalten eines ersten Satzes physikalischer Eigenschaften $S_M$ (50) von einem nichtvergänglichen computerlesbaren Speichermedium (36), wobei jede jeweilige physikalische Eigenschaft $S_{i,j}$ in $S_M$ eine physikalische Eigenschaft darstellt, die von einem Paar von Partikeln $(p_i, p_j)$ im Satz von $\{p_1, ..., p_N\}$ Partikeln (46) im Makromolekül geteilt wird;

(D) Auftragen der zweidimensionalen Koordinaten $(c_1,...c_N)$, nachdem die Austrittsbedingung (58) erreicht ist, als eine Vielzahl von Knoten (64) eines zweidimensionalen Graphs (62), wobei jeder Knoten einen Teil des Makromoleküls (44) darstellt; und

(E) Auftragen einer Vielzahl von Kanten (68) für den zweidimensionalen Graphen (62), wobei jede jeweilige Kante (68) in der Vielzahl von Kanten (68) ein zweidimensionales Koordinatenpaar $(c_i, c_j)$ im zweidimensionalen Graphen (62) verbindet, das einem Paar $(p_i, p_j)$ in einem Satz von $\{p_1, ..., p_N\}$ Partikeln (46) entspricht, und ein erstes Charakteristikum jeder jeweiligen Kante (68) in der Vielzahl von Kanten (68) wird bestimmt durch einen Wert von oder einen Typ von physikalischer Eigenschaft $S_{i,j}$ in $S_M$ für das Paar von Partikeln $(p_i, p_j)$ im Satz von $\{p_1, ..., p_N\}$ Partikeln (46), die dem zweidimensionalen Koordinatenpaar $(c_i, c_j)$ entsprechen, das durch die jeweilige Kante (68) verbunden ist.

14. Ein nichtvergängliches computerlesbares Speichermedium, angepasst zur Speicherung eines Visualisierungsmoduls zum Visualisieren physikalischer Eigenschaften eines Makromoleküls in zwei Dimensionen, basierend auf dreidimensionalen Daten der Makromoleküldaten (44), wobei die Makromoleküldaten (44) einen Satz von $\{p_1,..., p_N\}$ Partikeln (46) umfassen, wobei jedes jeweilige Partikel $p_i$ in dem Satz von Partikeln (46) eine unterschiedliche Vielzahl kovalent gebundener Atome in dem Makromolekül darstellt,

wobei das Visualisierungsmodul Anweisungen umfasst, die, wenn auf einem Computer ausgeführt, durchführt:

(A) Erhalten einen Satzes von N dreidimensionalen Koordinaten $\{x_1, ..., x_N\}$ (48) durch Röntgenkristallographie, Kernmagnetresonanz-spektroskopische Techniken, Elektronenmikroskopie, Modellieren oder von einem nichtvergänglichen computerlesbaren Speichermedium, wobei jedes jeweilige $x_i$ in $\{x_1, ..., x_N\}$ (48) einem Partikel $p_i$ im Satz von $\{p_1, ..., p_N\}$ Partikeln (46) entspricht und die Position von $p_i$ im dreidimensionalen Raum darstellt;

(B) Minimieren einer Kostenfunktion (56) umfassend den Fehler in einem Satz zweidimensionaler Koordinaten, die dem Satz von *N* dreidimensionalen Koordinaten entsprechen, unter Verwendung eines Minimierungsfunktionsmoduls (54):

$$E(c_1, c_2, ..., c_N) = \sum_{i<j}^{N} w_{ij} |\delta_{ij} - D(c_i, c_j)|^2$$

unter Verwendung des Satzes von *N* dreidimensionalen Koordinaten, wobei

*i* und *j* ganze Zahlen größer als Null sind,

$\delta_{ij}$ einer Entfernung zwischen einem Paar dreidimensionaler Koordinaten $x_i$ und $x_j$ in $\{x_1, ..., x_N\}$ (48) entspricht,

$E(c_1, c_2,...c_N)$ dem Fehler in dem Satz zweidimensionaler Koordinaten $(c_1,...c_N)$ entspricht, wobei jede zweidimensionale Koordinate $c_i$ in $(c_1,...c_N)$ eindeutig einer dreidimensionalen Koordinate $x_i$ in $\{x_1, ..., x_N\}$ (48) entspricht, so dass jedes jeweilige Partikel $p_i$ im Satz von $\{p_1, ..., p_N\}$ Partikeln (46) durch eine dreidimensionale Koordinate $x_i$ in $\{x_1, ..., x_N\}$ (48) und eine entsprechende zweidimensionale Koordinate $c_i$ im Satz zweidimensionaler Koordinaten $(c_1,...c_N)$ dargestellt wird,

$D(c_i, c_j)$ einer Entfernung zwischen den zweidimensionalen Koordinaten $c_i$ und $c_j$ in $(c_1,...c_N)$ entspricht, und $w_{ij}$ eine Gewichtung für das zweidimensionale Paar $(p_i, p_j)$ in einer Matrix von Gewichtungen ist, wobei die Matrix von Gewichtungen eine Gewichtung für jedes zweidimensionale Paar ($p_i$, $p_j$) im Satz von {$p_1$, ..., $p_N$} Partikeln (46) hat,

wobei das Minimieren der Kostenfunktion die Werte der Koordinaten des Satzes zweidimensionaler Koordinaten ($c_1...c_N$) unter Verwendung eines Verfeinerungsalgorithmus verfeinert, bis eine Austrittsbedingung (58) erreicht ist;

(C) Erhalten eines ersten Satzes physikalischer Eigenschaften $S_M$ (50) von einem nichtvergänglichen computerlesbaren Speichermedium (36), wobei jede jeweilige physikalische Eigenschaft $S_{i,j}$ in $S_M$ eine physikalische Eigenschaft darstellt, die von einem Paar von Partikeln ($p_i$, $p_j$) im Satz von {$p_1$, ..., $p_N$} Partikeln (46) im Makromolekül geteilt wird;

(D) Auftragen der zweidimensionalen Koordinaten ($c_1,...c_N$), nachdem die Austrittsbedingung (58) erreicht ist, als eine Vielzahl von Knoten (64) eines zweidimensionalen Graphs (62), wobei jeder Knoten einen Teil des Makromoleküls (44) darstellt; und

(E) Auftragen einer Vielzahl von Kanten (68) für den zweidimensionalen Graphen (62), wobei jede jeweilige Kante (68) in der Vielzahl von Kanten (68) ein zweidimensionales Koordinatenpaar ($c_i$, $c_j$) im zweidimensionalen Graphen (62) verbindet, das einem Paar ($p_i$, $p_j$) in einem Satz von {$p_1$, ..., $p_N$} Partikeln (46) entspricht, und ein erstes Charakteristikum jeder jeweiligen Kante (68) in der Vielzahl von Kanten (68) wird bestimmt durch einen Wert von oder einen Typ von physikalischer Eigenschaft $S_{i,j}$ in $S_M$ für das Paar von Partikeln ($p_i$, $p_j$) im Satz von {$p_1$, ..., $p_N$} Partikeln (46), die dem zweidimensionalen Koordinatenpaar ($c_i$, $c_j$) entsprechen, das durch die jeweilige Kante (68) verbunden ist.

15. Das computerimplementierte Visualisierungsverfahren aus Anspruch 1, wobei

(i) der Auftrageschritt (D) weiter Annehmen einer nutzereingegebenen manuellen Ortsänderung an einer zweidimensionalen Koordinaten $c_i$ in ($c_1,...c_N$) umfasst, oder

(ii) der Auftrageschritt (D) weiter Annehmen einer nutzereingegebenen Löschung einer zweidimensionalen Koordinaten $c_i$ in ($c_1,...c_N$) umfasst, oder

(iii) der Auftrageschritt (E) weiter Annehmen einer nutzereingegebenen manuellen Ortsänderung einer zweidimensionalen Koordinaten $c_i$ in ($c_1,...c_N$) oder einer Kante (68) in der Vielzahl von Kanten (68) umfasst, oder

(iv) der Auftrageschritt (E) weiter Annehmen einer nutzereingegebenen manuellen Ortsänderung einer zweidimensionalen Koordinaten $c_i$ in ($c_1...c_N$) und, als Antwort auf das Annehmen der manuellen Ortsänderung am $c_i$, Aktualisieren einer mit dem $c_i$ assoziierten Kante (68) umfasst, oder

(v) der Auftrageschritt (E) weiter Annehmen einer nutzereingegebenen manuellen Ortsänderung an einer Kante (68) in der Vielzahl von Kanten (68) und, als Antwort auf das Annehmen der manuellen Ortsänderung an der Kante (68), Aktualisieren eines mit dem $c_i$ ohne Nutzereingriff assoziierten $c_i$ in ($c_1,...c_N$) umfasst, oder

(vi) der Auftrageschritt (E) weiter Annehmen einer nutzereingegebenen Löschung einer zweidimensionalen Koordinaten $c_i$ in ($c_1,...c_N$) oder einer Kante (68) in der Vielzahl von Kanten (68) umfasst.

**Revendications**

1. Procédé implémenté par ordinateur pour visualiser des propriétés physiques d'une macromolécule en deux dimensions sur la base de données tridimensionnelles des données de macromolécule (44),

dans lequel les données de macromolécule (44) comprennent l'ensemble de {$p_1$, ..., $p_N$} particules (46), chaque particule $p_i$ respective dans l'ensemble de particules (46) représentant une pluralité différente d'atomes liés par covalence dans la macromolécule, le procédé étant réalisé sur un système d'ordinateur ayant au moins un processeur et une mémoire stockant au moins un programme pour une exécution par l'au moins un processeur pour réaliser le procédé, comprenant :

(A) l'obtention d'un ensemble de N coordonnées tridimensionnelles {$x_1$, ... , $x_N$} (48) par une cristallographie par rayons X, des techniques spectroscopiques par résonance magnétique nucléaire, une microscopie électronique, une modélisation ou à partir d'un support de stockage lisible par ordinateur non transitoire, dans lequel chaque $x_i$ respectif dans {$x_1$, ..., $x_N$} (48) correspond à une particule $p_i$ dans l'ensemble de {$p_1$, ..., $p_N$} particules (46) et représente la position de $p_i$ dans l'espace tridimensionnel ;

(B) la minimisation d'une fonction de coût (56) contenant l'erreur dans un ensemble de coordonnées bidimensionnelles correspondant à l'ensemble de N coordonnées tridimensionnelles à l'aide d'un module de fonction de minimisation (54) :

$$E(c_1, c_2, \ldots, c_N) = \sum_{i<j}^{N} w_{ij} \left| \delta_{ij} - D(c_i, c_j) \right|^2$$

utilisant l'ensemble de N coordonnées tridimensionnelles dans lequel,

i et j sont des nombres entiers supérieurs à zéro,

$\delta_{ij}$ correspond à une distance entre une paire de coordonnées tridimensionnelles $x_i$ et $x_j$ dans $\{x_1, \ldots, x_N\}$ (48),

$E(c_1, c_2, \ldots c_N)$ correspond à l'erreur dans l'ensemble de N coordonnées bidimensionnelles $(c_1, \ldots c_N)$ dans lequel chaque coordonnée bidimensionnelle $c_i$ dans $(c_1, \ldots c_N)$ correspond exclusivement à une coordonnée tridimensionnelle $x_i$ dans $\{x_1, \ldots, x_N\}$ (48) de sorte que chaque particule $p_i$ respective dans l'ensemble de $\{p_1, \ldots, p_N\}$ particules (46) soit représentée par une coordonnée tridimensionnelle $x_i$ dans $\{x_1, \ldots, x_N\}$ (48), et une coordonnée bidimensionnelle $c_i$ correspondante dans l'ensemble de coordonnées bidimensionnelles $(c_1, \ldots c_N)$,

$D(c_i, c_j)$ correspond à une distance entre les coordonnées bidimensionnelles $c_i$ et $c_j$ dans $(c_1, \ldots c_N)$, et

$w_{ij}$ est un poids pour la paire bidimensionnelle $(p_i, p_j)$ dans une matrice de poids, dans lequel la matrice de poids a un poids pour chaque paire bidimensionnelle $(p_i, p_j)$ dans l'ensemble de $\{p_1, \ldots, p_N\}$ particules (46), dans lequel la minimisation de la fonction de coût affine les valeurs de coordonnées de l'ensemble de coordonnées bidimensionnelles $(c_1, \ldots c_N)$ à l'aide d'un algorithme d'affinage jusqu'à ce qu'une condition de sortie (58) soit obtenue ;

(C) l'obtention d'un premier ensemble de propriétés physiques $S_M$ (50) à partir d'un support de stockage lisible par ordinateur non transitoire (36), chaque propriété physique $S_{i,j}$ respective dans $S_M$ représentant une propriété physique partagée par une paire de particules $(p_i, p_j)$ dans l'ensemble de $\{p_1, \ldots, p_N\}$ particules (46) dans la macromolécule ;

(D) le tracé des coordonnées bidimensionnelles $(c_1, \ldots c_N)$, après que la condition de sortie (58) est obtenue, sous forme de pluralité de noeuds (64) d'un graphique bidimensionnel (62), dans lequel chaque noeud représente une portion de la macromolécule (44) ; et

(E) le tracé d'une pluralité de contours (68) pour le graphique bidimensionnel (62), dans lequel chaque contour (68) respectif dans la pluralité de contours (68) relie une paire de coordonnées bidimensionnelles $(c_i, c_j)$ dans le graphique bidimensionnel (62) qui correspond à une paire de $(p_i, p_j)$ dans un ensemble de $\{p_1, \ldots, p_N\}$ particules (46), et

une première caractéristique de chaque contour (68) respectif dans la pluralité de contours (68) est déterminée par une valeur ou un type d'une propriété physique $S_{i,j}$ dans $S_M$ pour la paire de particules $(p_i, p_j)$ dans l'ensemble de $\{p_1, \ldots, p_N\}$ particules (46) correspondant à la paire de coordonnées bidimensionnelles $(c_i, c_j)$ qui est reliée par le contour (68) respectif.

2. Procédé de visualisation implémenté par ordinateur selon la revendication 1, dans lequel les poids sont définis sous la forme :

$$w_{ij} = \frac{1}{\delta_{ij}} \frac{1}{\sum_{k<l}^{N} \delta_{kl}}$$

dans lequel,

$\delta_{kl}$ correspond à une distance entre une paire de coordonnées tridimensionnelles $x_k$ et $x_1$ dans $\{x_1, \ldots, x_N\}$ (48) .

3. Procédé de visualisation implémenté par ordinateur selon la revendication 2, dans lequel l'algorithme d'affinage est une plus grande descente où la dérivée de la fonction de coût est exprimée sous la forme :

$$= \frac{-2}{\sum_{k<l}^{N} \delta_{kl}} \sum_{j, j \neq m}^{N} \frac{1}{\delta_{mj}} \left| \delta_{mj} - D(c_m, c_j) \right| \frac{(c_m - c_j)}{D(c_m, c_j)}.$$

dans lequel, $j, k, l$ et $m$ sont des nombres entiers supérieurs à zéro,

$\delta_{mj}$ correspond à une distance entre une paire de coordonnées tridimensionnelles $x_m$ et $x_j$ dans $\{x_1, \ldots, x_N\}$ (48),

$D(c_m, c_j)$ correspond à une distance entre les coordonnées bidimensionnelles $c_m$ et $c_j$ dans $(c_1, \ldots c_N)$, et

$\delta_{k,1}$ correspond à une distance entre une paire de coordonnées tridimensionnelles $x_k$ et $x_1$ dans $\{x_1, \ldots, x_N\}$ (48) .

4. Procédé de visualisation implémenté par ordinateur selon l'une quelconque des revendications 1 à 3, dans lequel la macromolécule est un polypeptide,

chaque particule $p_i$ dans l'ensemble de $\{p_1, ..., p_N\}$ particules (46) représente un résidu dans le polypeptide, et chaque $x_i$ respectif dans $\{x_1, ..., x_N\}$ (48) correspond aux coordonnées tridimensionnelles du carbone $C_\alpha$ de chaque résidu respectif représenté par le $p_i$ dans l'ensemble de $\{p_1, ..., p_N\}$ particules (46) qui correspond au $x_i$ respectif.

5. Procédé de visualisation implémenté par ordinateur selon l'une quelconque des revendications 1 à 3, dans lequel la macromolécule est un acide polynucléique, un acide polyribonucléique, un polysaccharide ou un polypeptide.

6. Procédé de visualisation implémenté par ordinateur selon l'une quelconque des revendications 1 à 3, dans lequel

(i) la macromolécule est un acide polynucléique et chaque particule $p_i$ respective dans l'ensemble de $\{p_1, ..., p_N\}$ particules (46) représente un résidu d'acide nucléique dans l'acide polynucléique, ou
(ii) la macromolécule est un acide polyribonucléique et chaque particule $p_i$ respective dans l'ensemble de $\{p_1, p_N\}$ particules (46) représente un résidu d'acide ribonucléique dans l'acide polyribonucléique, ou
(iii) la macromolécule est un polysaccharide et chaque particule $p_i$ respective dans l'ensemble de $\{p_1, ..., p_N\}$ particules (46) représente un motif monosaccharide ou un motif disaccharide dans le polysaccharide, ou
(iv) la macromolécule est un polypeptide et chaque particule $p_i$ respective dans l'ensemble de $\{p_1, ..., p_N\}$ particules (46) représente un résidu dans le polypeptide, ou
(v) la macromolécule est un tensioactif, un composé organométallique, un fullerène ou un polymère.

7. Procédé de visualisation implémenté par ordinateur selon l'une quelconque des revendications 1 à 6, dans lequel la propriété physique représentée par $S_{i,j}$ partagée par chaque paire respective de particules $(p_i, p_j)$ dans un ensemble de $\{p_1, ..., p_N\}$ particules (46) dans la macromolécule, est

(i) une présence d'une liaison covalente entre un premier atome dans la pluralité d'atomes représentés par la particule $p_i$ et un second atome dans la pluralité d'atomes représentés par la particule $p_j$, ou
(ii) une présence d'une liaison hydrogène entre un premier atome dans la pluralité d'atomes représentés par la particule $p_i$ et un second atome dans la pluralité d'atomes représentés par la particule $p_j$, ou
(iii) une présence d'un contact carbone-carbone, d'un contact carbone-soufre, d'un contact soufre-soufre, d'un contact carbone-azote ou d'un contact carbone-oxygène entre un premier atome dans la pluralité d'atomes représentés par la particule $p_i$ et un second atome dans la pluralité d'atomes représentés par la particule $p_j$ ou
(iv) une interaction $\pi$-$\pi$ ou une interaction $\pi$-cation entre une première portion de la pluralité d'atomes représentés par la particule $p_i$ et un second atome dans la pluralité d'atomes représentés par la particule $p_j$.

8. Procédé de visualisation implémenté par ordinateur selon l'une quelconque des revendications 1 à 7, dans lequel la première caractéristique de chaque contour respectif à l'étape (E) est

(i) l'épaisseur de ligne et une épaisseur de ligne d'un contour dans la pluralité de contours dans le graphique bidimensionnel (62) sont déterminées par une valeur ou un type de la propriété physique dans $S_M$ pour chaque paire respective de particules $(p_i, p_j)$ dans l'ensemble de $\{p_1, ..., p_N\}$ particules (46) correspondant à chaque paire de coordonnées bidimensionnelles $(c_i, c_j)$ respective qui est reliée par le contour, ou
(ii) la coloration de ligne et une couleur d'un contour dans la pluralité de contours dans le graphique bidimensionnel (62) sont déterminées par une valeur ou un type de la propriété physique dans $S_M$ pour chaque paire respective de particules $(p_i, p_j)$ dans l'ensemble de $\{p_1, ... , p_N\}$ particules (46) correspondant à chaque paire de coordonnées bidimensionnelles $(c_i, c_j)$ respective qui est reliée par le contour, ou
(iii) la formation de motifs de ligne et un motif d'un contour dans la pluralité de contours dans le graphique bidimensionnel (62) sont déterminés par une valeur ou un type de la propriété physique dans $S_M$ pour chaque paire respective de particules $(p_i, p_j)$ dans l'ensemble de $\{p_1, ..., p_N\}$ particules (46) correspondant à chaque paire de coordonnées bidimensionnelles $(c_i, c_j)$ respective qui est reliée par le contour.

9. Procédé de visualisation implémenté par ordinateur selon l'une quelconque des revendications 1 à 8, le procédé comprenant en outre :
l'obtention d'un second ensemble de propriétés physiques $K_M$ (52), chaque propriété physique $k_i$ dans $K_M$ (52) représentant une propriété physique d'une particule $p_i$ correspondante dans l'ensemble de $\{p_1, ..., p_N\}$ particules (46), et dans lequel une seconde caractéristique de chaque noeud (64) respectif dans la pluralité de noeuds (64) dans le graphique bidimensionnel (62) est déterminée par une valeur ou un type de la propriété physique de la particule $p_i$ correspondante dans $K_M$ (52).

**10.** Procédé de visualisation implémenté par ordinateur selon la revendication 9, dans lequel la propriété physique $k_i$ correspond à

(i) une superficie accessible ou une surface à exclusion de solvant de la pluralité d'atomes dans la macromolécule qui sont représentés par la particule $p_i$ correspondante, ou
(ii) une charge électrique, une hydrophobicité, un caractère hydrophile, une polarité, une aromaticité, un poids moléculaire ou un volume de la pluralité d'atomes dans la macromolécule qui sont représentés par la particule $p_i$ correspondante.

**11.** Procédé de visualisation implémenté par ordinateur selon la revendication 9, dans lequel la seconde caractéristique est

(i) l'ombrage et une luminosité du noeud (64) respectif sont déterminés par une valeur ou un type de la propriété physique de la particule $p_i$ correspondante dans $K_M$ (52), ou
(ii) la taille et une taille du noeud (64) respectif sont déterminées par une valeur ou un type de la propriété physique de la particule $p_i$ correspondante dans $K_M$ (52), ou
(iii) la couleur et une couleur du noeud (64) respectif sont déterminées par une valeur ou un type de la propriété physique de la particule $p_i$ correspondante dans $K_M$ (52).

**12.** Procédé de visualisation implémenté par ordinateur selon l'une quelconque des revendications 1 à 11, dans lequel la condition de sortie est obtenue lorsqu'un nombre maximal prédéterminé d'itérations de l'algorithme d'affinage ont été calculées.

**13.** Système d'ordinateur configuré pour visualiser des propriétés physiques d'une macromolécule en deux dimensions sur la base de données tridimensionnelles des données de macromolécule (44), dans lequel les données de macromolécule (44) comprennent l'ensemble de $\{p_1, ..., p_N\}$ particules (46), chaque particule $p_i$ respective dans l'ensemble de particules (46) représentant une pluralité différente d'atomes liés par covalence dans la macromolécule, le système d'ordinateur comprenant :

au moins un processeur ; et
une mémoire stockant au moins un programme d'ordinateur,
dans lequel l'au moins un processeur est configuré pour exécuter l'au moins un programme comprenant des instructions pour :

(A) obtenir un ensemble de N coordonnées tridimensionnelles $\{x_1, ..., x_N\}$ (48) par une cristallographie par rayons X, des techniques spectroscopiques par résonance magnétique nucléaire, une microscopie électronique, une modélisation ou à partir d'un support de stockage lisible par ordinateur non transitoire, dans lequel chaque $x_i$ respectif dans $\{x_1, ..., x_N\}$ (48) correspond à une particule $p_i$ dans l'ensemble de $\{p_1, ..., p_N\}$ particules (46) et représente la position de $p_i$ dans l'espace tridimensionnel ;
(B) minimiser une fonction de coût (56) contenant l'erreur dans un ensemble de coordonnées bidimensionnelles correspondant à l'ensemble de N coordonnées tridimensionnelles à l'aide d'un module de fonction de minimisation (54) :

$$E(c_1, c_2, ..., c_N) = \sum_{i<j}^{N} w_{ij} \left| \delta_{ij} - D(c_i, c_j) \right|^2$$

utilisant l'ensemble de N coordonnées tridimensionnelles dans lequel,
i et j sont des nombres entiers supérieurs à zéro,
$\delta_{ij}$ correspond à une distance entre une paire de coordonnées tridimensionnelles $x_i$ et $x_j$ dans $\{x_1, ... , x_N\}$ (48),
$E(c_1, c_2, ... c_N)$ correspond à l'erreur dans l'ensemble de coordonnées bidimensionnelles $(c_1, ... c_N)$ dans lequel chaque coordonnée bidimensionnelle $c_i$ dans $(c_1, ... c_N)$ correspond exclusivement à une coordonnée tridimensionnelle $x_i$ dans $\{x_1, ..., x_N\}$ (48) de sorte que chaque particule $p_i$ respective dans l'ensemble de $\{p_1, ..., p_N\}$ particules (46) soit représentée par une coordonnée tridimensionnelle $x_i$ dans $\{x_1, ..., x_N\}$ (48), et une coordonnée bidimensionnelle $c_i$ correspondante dans l'ensemble de coordonnées bidimensionnelles $(c_1, ... c_N)$ ,

$D(c_i, c_j)$ correspond à une distance entre les coordonnées bidimensionnelles $c_i$ et $c_j$ dans $(c_1, ... c_N)$, et $w_{ij}$ est un poids pour la paire bidimensionnelle $(p_i, p_j)$ dans une matrice de poids, dans lequel la matrice de poids a un poids pour chaque paire bidimensionnelle $(p_i, p_j)$ dans l'ensemble de $\{p_1, ..., p_N\}$ particules (46),

dans lequel la minimisation de la fonction de coût affine les valeurs de coordonnées de l'ensemble de coordonnées bidimensionnelles $(c_1, ... c_N)$ à l'aide d'un algorithme d'affinage jusqu'à ce qu'une condition de sortie (58) soit obtenue ;

(C) obtenir un premier ensemble de propriétés physiques $S_M$ (50) à partir d'un support de stockage lisible par ordinateur non transitoire (36), chaque propriété physique $S_{i,j}$ respective dans $S_M$ représentant une propriété physique partagée par une paire de particules $(p_i, p_j)$ dans l'ensemble de $\{p_1, ..., p_N\}$ particules (46) dans la macromolécule ;

(D) tracer les coordonnées bidimensionnelles $(c_1, ... c_N)$, après que la condition de sortie (58) est obtenue, sous forme de pluralité de noeuds (64) d'un graphique bidimensionnel (62), dans lequel chaque noeud représente une portion de la macromolécule (44) ;

(E) tracer une pluralité de contours (68) pour le graphique bidimensionnel (62), dans lequel chaque contour (68) respectif dans la pluralité de contours (68) relie une paire de coordonnées bidimensionnelles $(c_i, c_j)$ dans le graphique bidimensionnel (62) qui correspond à une paire de $(p_i, p_j)$ dans un ensemble de $\{p_1, ..., p_N\}$ particules (46), et

une première caractéristique de chaque contour (68) respectif dans la pluralité de contours (68) est déterminée par une valeur ou un type d'une propriété physique $S_{i,j}$ dans $S_M$ pour la paire de particules $(p_i, p_j)$ dans l'ensemble de $\{p_1, ..., p_N\}$ particules (46) correspondant à la paire de coordonnées bidimensionnelles $(c_i, c_j)$ qui est reliée par le contour (68) respectif.

14. Support de stockage lisible par ordinateur non transitoire adapté pour stocker un module de visualisation pour visualiser des propriétés physiques d'une macromolécule en deux dimensions sur la base de données tridimensionnelles des données de macromolécule (44), dans lequel les données de macromolécule (44) comprennent l'ensemble de $\{p_1, ..., p_N\}$ particules (46), chaque particule $p_i$ respective dans l'ensemble de particules (46) représentant une pluralité différente d'atomes liés par covalence dans la macromolécule,

dans lequel le module de visualisation comprend des instructions qui, lorsqu'elles sont exécutées sur un ordinateur, réalisent :

(A) l'obtention d'un ensemble de $N$ coordonnées tridimensionnelles $\{x_1, ..., x_N\}$ (48) par une cristallographie par rayons X, des techniques spectroscopiques par résonance magnétique nucléaire, une microscopie électronique, une modélisation ou à partir d'un support de stockage lisible par ordinateur non transitoire, dans lequel chaque $x_i$ respectif dans $\{x_1, ..., x_N\}$ (48) correspond à une particule $p_i$ dans l'ensemble de $\{p_1, ..., p_N\}$ particules (46) et représente la position de $p_i$ dans l'espace tridimensionnel ;

(B) la minimisation d'une fonction de coût (56) contenant l'erreur dans un ensemble de coordonnées bidimensionnelles correspondant à l'ensemble de $N$ coordonnées tridimensionnelles à l'aide d'un module de fonction de minimisation (54) :

$$E(c_1, c_2, ..., c_N) = \sum_{i<j}^{N} w_{ij} \left| \delta_{ij} - D(c_i, c_j) \right|^2$$

utilisant l'ensemble de $N$ coordonnées tridimensionnelles dans lequel,
$i$ et $j$ sont des nombres entiers supérieurs à zéro,
$\delta_{ij}$ correspond à une distance entre une paire de coordonnées tridimensionnelles $x_i$ et $x_j$ dans $\{x_1, ..., x_N\}$ (48),
$E(c_1, c_2, ... c_N)$ correspond à l'erreur dans l'ensemble de N coordonnées bidimensionnelles $(c_1, ... c_N)$ dans lequel chaque coordonnée bidimensionnelle $c_i$ dans $(c_1, ... c_N)$ correspond exclusivement à une coordonnée tridimensionnelle $x_i$ dans $\{x_1, ..., x_N\}$ (48) de sorte que chaque particule $p_i$ respective dans l'ensemble de $\{p_1, ..., p_N\}$ particules (46) soit représentée par une coordonnée tridimensionnelle $x_i$ dans $\{x_1, ..., x_N\}$ (48), et une coordonnée bidimensionnelle $c_i$ correspondante dans l'ensemble de coordonnées bidimensionnelles $(c_1, ... c_N)$,
$D(c_i, c_j)$ correspond à une distance entre les coordonnées bidimensionnelles $c_i$ et $c_j$ dans $(c_1, ... c_N)$, et $w_{ij}$ est un poids pour la paire bidimensionnelle $(p_i, p_j)$ dans une matrice de poids, dans lequel la matrice de poids a un poids pour chaque paire bidimensionnelle $(p_i, p_j)$ dans l'ensemble de $\{p_1, ..., p_N\}$ particules (46),

dans lequel la minimisation de la fonction de coût affine les valeurs de coordonnées de l'ensemble de coordonnées bidimensionnelles ($c_1$, ... $c_N$) à l'aide d'un algorithme d'affinage jusqu'à ce qu'une condition de sortie (58) soit obtenue ;

(C) l'obtention d'un premier ensemble de propriétés physiques $S_M$ (50) à partir d'un support de stockage lisible par ordinateur non transitoire (36), chaque propriété physique $S_{i,j}$ respective dans $S_M$ représentant une propriété physique partagée par une paire de particules ($p_i$, $p_j$) dans l'ensemble de $\{p_1, ..., p_N\}$ particules (46) dans la macromolécule ;

(D) le tracé des coordonnées bidimensionnelles ($c_1$, ... $c_N$), après que la condition de sortie (58) est obtenue, sous forme de pluralité de noeuds (64) d'un graphique bidimensionnel (62), dans lequel chaque noeud représente une portion de la macromolécule (44) ;

(E) le tracé d'une pluralité de contours (68) pour le graphique bidimensionnel (62), dans lequel chaque contour (68) respectif dans la pluralité de contours (68) relie une paire de coordonnées bidimensionnelles ($c_i$, $c_j$) dans le graphique bidimensionnel (62) qui correspond à une paire de ($p_i$, $p_j$) dans un ensemble de $\{p_1, ..., p_N\}$ particules (46), et

une première caractéristique de chaque contour (68) respectif dans la pluralité de contours (68) est déterminée par une valeur ou un type d'une propriété physique $S_{i,j}$ dans $S_M$ pour la paire de particules ($p_i$, $p_j$) dans l'ensemble de $\{p_1, ..., p_N\}$ particules (46) correspondant à la paire de coordonnées bidimensionnelles ($c_i$, $c_j$) qui est reliée par le contour (68) respectif.

**15.** Procédé de visualisation implémenté par ordinateur selon la revendication 1, dans lequel

(i) l'étape de tracé (D) comprend en outre l'acceptation d'un changement spatial manuel entré par l'utilisateur à une coordonnée bidimensionnelle $c_i$ dans ($c_1$, ... $c_N$), ou

(ii) l'étape de tracé (D) comprend en outre l'acceptation d'une suppression entrée par l'utilisateur d'une coordonnée bidimensionnelle $c_i$ dans ($c_1$, ... $c_N$), ou

(iii) l'étape de tracé (E) comprend en outre l'acceptation d'un changement spatial manuel entré par l'utilisateur d'une coordonnée bidimensionnelle $c_i$ dans ($c_1$, ... $c_N$) ou d'un contour (68) dans la pluralité de contours (68), ou

(iv) l'étape de tracé (E) comprend en outre l'acceptation d'un changement spatial manuel entré par l'utilisateur d'une coordonnée bidimensionnelle $c_i$ dans ($c_1$, ... $c_N$) et, en réponse à l'acceptation du changement spatial manuel à la $c_i$, la mise à jour d'un contour (68) associé à la $c_i$, ou

(v) l'étape de tracé (E) comprend en outre l'acceptation d'un changement spatial manuel entré par l'utilisateur à un contour (68) dans la pluralité de contours (68) et, en réponse à l'acceptation du changement spatial manuel au contour (68), la mise à jour d'une $c_i$ dans ($c_1$, ... $c_N$) associée à la $c_i$ sans l'intervention de l'utilisateur, ou

(vi) l'étape de tracé (E) comprend en outre l'acceptation d'une suppression entrée par l'utilisateur d'une coordonnée bidimensionnelle $c_i$ dans ($c_1$, ... $c_N$) ou d'un contour (68) dans la pluralité de contours (68).

11

10

Wide area network  34

| Power Source | CPU | Communications Circuitry |

24

22

20

30

32

36

| Operating System | 40 |
|---|---|
| File system | 41 |
| Communication module | 42 |
| User interface module | 43 |
| Molecule | 44 |
|   Set of {$p_1$, ..., $p_N$} particles | 46 |
|   Set of three-dimensional points {$x_1$, ..., $x_N$} | 48 |
|   First set of physical properties $S_M$ | 50 |
|   Optional second set of physical properties $K_M$ | 52 |
| Minimization function module | 54 |
|   Cost function | 56 |
|   Exit condition | 58 |
| Molecule plotting module | 60 |
| Two-dimensional graph | 62 |
|   Node 1 | 64-1 |
|   Optional node 1 characteristic | 66-1 |
|   ⋮ | |
|   Node M | 64-M |
|   Optional node M characteristic | 66-M |
|   Edge 1 | 68-1 |
|   Edge 1 characteristic | 70-1 |
|   ⋮ | |
|   Edge N | 68-N |
|   Edge N characteristic | 70-N |
| Interactive adjustment module | 72 |

⋮

26

28

72

12

Controller

14

Fig. 1

```
                                                                    ⌐ 202
┌─────────────────────────────────────────────────────────────────────┐
│ Obtain, for a complex molecule comprising the set of {p₁, ..., pₙ} particles, a │
│ set of N three-dimensional points {x₁, ..., xₙ}, where each particle pᵢ in the set │
│ of particles represents a different plurality of covalently bound atoms in the │
│ molecule and each respective xᵢ in {x₁, ..., xₙ} corresponds to a pᵢ in {p₁, ...,│
│ pₙ} and represents the position of pᵢ in three-dimensional space. │
└─────────────────────────────────────────────────────────────────────┘
```

Obtain, for a complex molecule comprising the set of $\{p_1, ..., p_N\}$ particles, a set of $N$ three-dimensional points $\{x_1, ..., x_N\}$, where each particle $p_i$ in the set of particles represents a different plurality of covalently bound atoms in the molecule and each respective $x_i$ in $\{x_1, ..., x_N\}$ corresponds to a $p_i$ in $\{p_1, ..., p_N\}$ and represents the position of $p_i$ in three-dimensional space.

⌐ 204

Minimize a cost function containing the error in a set of two-dimensional points $(c_1, ..., c_N)$, where each $c_i$ in $(c_1, ..., c_N)$ corresponds to a three-dimensional point $x_i$ in $\{x_1, ..., x_N\}$, until an exit condition is achieved.

⌐ 206

Obtain a first set of physical properties $S_M$, each physical property $s_{i,j}$ in $S_M$ representing a physical property shared by a pair of particles $(p_i, p_j)$ in $\{p_1, ..., p_N\}$.

⌐ 208

Optionally, obtain a second set of physical properties $K_M$, each physical property $k_i$ in $K_M$ representing a physical property of a particle $p_i$ in $\{p_1, ..., p_N\}$.

⌐ 210

Plot $(c_1, ..., c_N)$, after the exit condition is achieved, as a plurality of nodes of a two-dimensional graph. Optionally, a second characteristic of a node in the plurality of nodes in the graph is determined by a value of or a type of the physical property of the corresponding particle $p_i$ in $K_M$.

⌐ 212

Plot a plurality of edges for the two-dimensional graph, where each respective edge in the plurality of edges connects a two-dimensional coordinate pair $(c_i, c_j)$ in the graph that corresponds to a pair of particles $(p_i, p_j)$ in $\{p_1, ..., p_N\}$, and a first characteristic of each respective edge in the plurality of edges is determined by a physical property $s_{i,j}$ in $S_M$ for the pair of particles $(p_i, p_j)$ in $\{p_1, ..., p_N\}$ corresponding to the two-dimensional coordinate pair $(c_i, c_j)$ that is connected by the respective edge.

# Fig. 2

Fig. 3

Fig. 4

Fig. 5

Fig. 6

EP 2 828 779 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6125235 A
- WO 2011047684 A
- WO 2010000268 A

### Non-patent literature cited in the description

- **JENKINS.** Glossary of Basic Terms in Polymer Science. *Pure Appl. Chem.,* 1996, vol. 68 (12), 2287-2311
- **PAINTER.** Fundamentals of Polymer Science. CRC Press, 1997, 14
- **RUBINSTEIN et al.** Polymer physics. Oxford University Press, 2003, 6
- **SIMON et al.** *Proceedings of the National Academy of Sciences USA,* 1992, vol. 89, 9367
- **CHIN et al.** *Science,* 2003, vol. 301, 964
- **CHIN et al.** *Chemistry & Biology,* 2003, vol. 10, 511
- **BERINDE.** *Novi SAD J. Math,* 1997, vol. 27, 19-26
- **BAKER ; HUBBARD.** *Prog. Biophy. Mol. Biol.,* 1984, vol. 44, 97-179
- **BROCCHIERI ; KARLIN.** *PNAS,* 1994, vol. 91 (20), 9297-9301
- **LEE ; RICHARDS.** *J. Mol. Biol.,* 1971, vol. 55 (3), 379-400
- **SHRAKE ; RUPLEY.** *J. Mol. Biol.,* 1973, vol. 79 (2), 351-371
- **RICHARDS.** *Annu Rev Biophys Bioeng,* 1977, vol. 6, 151-176
- **CONNOLLY.** *J Mol Graphics,* 1992, vol. 11 (2), 139-141